(19) **Europäisches Patentamt / European Patent Office / Office européen des brevets**

(11) **EP 2 171 057 B1**

(12) **EUROPEAN PATENT SPECIFICATION**

(45) Date of publication and mention
of the grant of the patent:
**30.09.2015 Bulletin 2015/40**

(51) Int Cl.:
**C12N 15/10** (2006.01)

(21) Application number: **08768190.4**

(22) Date of filing: **06.06.2008**

(86) International application number:
**PCT/US2008/007114**

(87) International publication number:
**WO 2008/153935 (18.12.2008 Gazette 2008/51)**

(54) **METHODS FOR IMPROVING PROTEIN PROPERTIES**

VERFAHREN ZUR VERBESSERUNG VON PROTEINEIGENSCHAFTEN

PROCÉDÉS D'AMÉLIORATION DES PROPRIÉTÉS DE PROTÉINES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **06.06.2007 US 933331 P**
**06.06.2007 US 933307 P**
**06.06.2007 US 933312 P**

(43) Date of publication of application:
**07.04.2010 Bulletin 2010/14**

(73) Proprietor: **Danisco US Inc.**
**Palo Alto, CA 94304 (US)**

(72) Inventors:
• **AEHLE, Wolfgang**
**Palo Alto, California 94304 (US)**
• **CASCAO-PEREIRA, Luis Gustavo**
**Palo Alto, California 94304 (US)**
• **KELLIS, JR., James T.**
**Palo Alto, California 94304 (US)**
• **SHAW, Andrew**
**Palo Alto, California 94304 (US)**

(74) Representative: **Casley, Christopher Stuart et al**
**Mewburn Ellis LLP**
**City Tower**
**40 Basinghall Street**
**London EC2V 5DE (GB)**

(56) References cited:
**EP-A- 0 995 801**    **EP-A- 1 553 174**
**WO-A-2007/044993**    **WO-A1-95/09909**
**WO-A2-2007/044993**

• **STRAUSBERG SUSAN L ET AL: "Directed coevolution of stability and catalytic activity in calcium-free subtilisin" BIOCHEMISTRY, vol. 44, no. 9, March 2005 (2005-03), pages 3272-3279, XP002506364 ISSN: 0006-2960**
• **REETZ MANFRED T ET AL: "Iterative saturation mutagenesis (ISM) for rapid directed evolution of functional enzymes" NATURE PROTOCOLS, NATURE PUBLISHING GROUP, UK, vol. 2, no. 4, 12 April 2007 (2007-04-12), pages 891-903, XP001538725 ISSN: 1750-2799**
• **CHICA ET AL: "Semi-rational approaches to engineering enzyme activity: combining the benefits of directed evolution and rational design" CURRENT OPINION IN BIOTECHNOLOGY, LONDON, GB, vol. 16, no. 4, 1 August 2005 (2005-08-01), pages 378-384, XP005006161 ISSN: 0958-1669**
• **MANSFELD JOHANNA ET AL: "The stability of engineered thermostable neutral proteases from Bacillus stearothermophilus in organic solvents and detergents." BIOTECHNOLOGY AND BIOENGINEERING 1 JUL 2007, vol. 97, no. 4, 12 December 2006 (2006-12-12), - 1 July 2007 (2007-07-01) pages 672-679, XP002500468 on-line 12-12-2006 ISSN: 0006-3592**
• **BOMMARIUS ET AL: "High-throughput screening for enhanced protein stability" CURRENT OPINION IN BIOTECHNOLOGY, LONDON, GB, vol. 17, no. 6, 24 November 2006 (2006-11-24), pages 606-610, XP005779244 ISSN: 0958-1669**

**(Cont. next page)**

- BOTT R ET AL: "The role of high-resolution structural studies in the development of commercial enzymes." CURRENT OPINION IN BIOTECHNOLOGY AUG 1999, vol. 10, no. 4, August 1999 (1999-08), pages 391-397, XP002500470 ISSN: 0958-1669
- MORLEY K L ET AL: "Improving enzyme properties: when are closer mutations better?" TRENDS IN BIOTECHNOLOGY, ELSEVIER PUBLICATIONS, CAMBRIDGE, GB, vol. 23, no. 5, 1 May 2005 (2005-05-01), pages 231-237, XP004872218 ISSN: 0167-7799
- BORNSCHEUER U T ET AL: "Optimizing lipases and related enzymes for efficient application" TRENDS IN BIOTECHNOLOGY, ELSEVIER PUBLICATIONS, CAMBRIDGE, GB, vol. 20, no. 10, 1 October 2002 (2002-10-01), pages 433-437, XP004379929 ISSN: 0167-7799
- GUPTA R ET AL: "Bacterial alkaline proteases: Molecular approaches and industrial applications" APPLIED MICROBIOLOGY AND BIOTECHNOLOGY, SPRINGER VERLAG, BERLIN, DE, vol. 59, no. 1, 1 June 2002 (2002-06-01), pages 15-32, XP002243519 ISSN: 0175-7598
- PATRICK ET AL: "Strategies and computational tools for improving randomized protein libraries" BIOMOLECULAR ENGINEERING, ELSEVIER, NEW YORK, NY, US, vol. 22, no. 4, 1 October 2005 (2005-10-01), pages 105-112, XP005042322 ISSN: 1389-0344
- SELLAMI-KAMOUN ALYA ET AL: "Stability of thermostable alkaline protease from Bacillus licheniformis RP1 in commercial solid laundry detergent formulations." MICROBIOLOGICAL RESEARCH 2008, vol. 163, no. 3, 2008, - 26 July 2006 (2006-07-26) pages 299-306, XP002505821 e-pub ISSN: 0944-5013
- Samantha S. Strickler ET AL: "Protein Stability and Surface Electrostatics: A Charged Relationship +", Biochemistry, vol. 45, no. 9, 1 March 2006 (2006-03-01), pages 2761-2766, XP055103244, ISSN: 0006-2960, DOI: 10.1021/bi0600143

## Description

### FIELD OF THE INVENTION

The present invention provides methods for engineering enzymes to optimize their A performance under certain environmental conditions of interest. In some embodiments, the present invention provides methods for engineering enzymes to optimize their catalytic activity under particular environmental conditions. In some preferred embodiments, the present invention provides methods for engineering enzymes to optimize their catalytic activity and/or stability under adverse environmental conditions. In some preferred embodiments, the present invention provides methods for engineering enzymes to optimize their stability, particularly under adverse environmental conditions. In some preferred embodiments, the present invention provides methods for altering the net surface charge and/or surface charge distribution of enzymes (e.g., metalloproteases) to obtain enzyme variants that demonstrate improved performance and/or stability in detergent formulations as compared to the starting or parent enzyme.

### BACKGROUND OF THE INVENTION

The properties of proteins functioning outside their natural milieu are often suboptimal. For instance, enzymes (e.g., proteases, lipases, amylases, cellulases, etc.) are frequently used for cleaning stains from fabric in laundry detergents, which typically include a complex combination of active ingredients. In fact, most cleaning products include a surfactant system, bleaching agents, builders, suds suppressors, soil-suspending agents, soil-release agents, optical brighteners, softening agents, dispersants, dye transfer inhibition compounds, abrasives, bactericides, and perfumes, as well as enzymes for cleaning. Thus despite the complexity of current detergents, there are many stains that are difficult to completely remove, due in part to suboptimal enzyme performance. Despite much research in enzyme development, there remains a need in the art for methods to engineer proteins for particular uses and conditions. Indeed, there remains a need in the art for methods to rapidly and systematically tailor electrostatic properties to optimize A enzyme performance in commercial applications. In particular, there remains a need in the art for methods to engineer industrially useful enzymes, including but not limited to lipases, amylases, cutinases, mannanases, oxidoreductases, cellulases, pectinases, proteases, and other enzymes, in order to provide improved activity, stability, and solubility in cleaning solutions.

### SUMMARY OF THE INVENTION

The present invention provides methods for engineering enzymes to optimize their performance under certain environmental conditions of interest. In some embodiments, the present invention provides methods for engineering enzymes to optimize their catalytic activity under particular environmental conditions. In some preferred embodiments, the present invention provides methods for engineering enzymes to optimize their catalytic activity and/or stability under adverse environmental conditions. In some preferred embodiments, the present invention provides methods for engineering enzymes to optimize their stability, particularly under adverse environmental conditions. In some preferred embodiments, the present invention provides methods for altering the net surface charge and/or surface charge distribution of enzymes (e.g., metalloproteases) to obtain enzyme variants that demonstrate improved performance and/or stability in detergent formulations as compared to the starting or parent enzyme.

The present invention provides methods for producing improved enzyme variants, comprising: testing a plurality of singly-substituted enzyme variants in a first test of a first property and a second test of a second property, wherein the property of a parent enzyme is given a value of 1.0 in each test, a favorable first or second property has a value greater than 1.0, and an unduly unfavorable first or second property has a value less than 0.80 ; identifying a substitution in at least one of the singly-substituted enzyme variants that is associated with a favorable first property and which is not associated with an unduly unfavorable second property; identifying a substitution in at least one of the singly-substituted enzyme variants that is associated with a favorable second property and which is not associated with an unduly unfavorable first property; introducing the substitution from the previous steps into a protein to yield a multiply-substituted enzyme variant; testing the multiply-substituted enzyme variant in the first and the second test, wherein said multiply substituted enzyme variant is an improved enzyme variant, and wherein at least one of the substitutions comprises a net charge change of 0, -1, or -2 relative to the parent enzyme, and at least one of the substitutions comprises a net charge change of +1 or +2 relative to the parent enzyme, thereby defining a charge-balanced multiply-substituted enzyme variant. An improved enzyme variant is a variant which achieves a value of greater than 1.0 in both said first and second tests, or a value of greater than 1.0 in the first test and a value of 0.80 to 1.0 in the second test. When said parent enzyme is a protease, said first property is stability and said second property is wash performance. When said parent enzyme is an amylase, said first property is protein expression and said second property is enzymatic activity. In some further embodiments, the methods further comprise producing the improved enzyme variant(s).

In some embodiments, the first and second properties are negatively correlated. In some preferred embodiments, stability comprises stability in a detergent composition and wash performance comprises blood milk ink (BMI) wash performance in detergent. In some additional embodiments, a favorable first or second property has a value greater than 1.2. In some further embodiments, an unduly unfavorable first or second property has a value less than 0.60. In yet further embodiments, an unduly unfavorable first or second property has a value less than 0.40. In additional embodiments wash performance is tested in a powder or liquid detergent composition having a pH of between 5 and 12. In some further embodiments wash performance is tested in cold water liquid detergent having a basic pH.

In some embodiments, the parent enzyme is a protease selected from neutral metalloproteases, and serine protesases. In some embodiments the serine protease is subtilisin. In some embodiments, the neutral metalloprotease is a neutral metalloprotease obtained from a member of the family Bacillaceae. In some further embodiments, the neutral metalloprotease is B. subtilis NprE. In some embodiments the parent enzyme is an alpha amylase obtained from a member of the family Bacillaceae.

In some preffered embodiments, the substitutions are in positions in the parent enzyme having a solvent accessible surface (SAS) of greater than about 25%. In still further embodiments, the substitutions are in positions in the parent enzyme having a solvent accessible surface (SAS) of greater than 50% or greater than 65%.

In some particularly preferred embodiments, the parent enzyme is a wild-type enzyme.

The present invention also provides cleaning compositions comprising the improved enzyme variant(s) produced according to methods set forth herein.

## BRIEF DESCRIPTION OF THE DRAWINGS

FIG. 1A shows the relative specific BODIPY-starch hydrolysis activity versus shake tube expression for an AmyS-S242Q combinatorial charge library (CCL).

FIG. 1B shows the relative rice starch microswatch cleaning activity in TIDE 2x versus shake tube expression for an AmyS-S242Q CCL.

FIG. 2A shows the relative shake tube expression versus relative net charge change for an AmyS-S242Q CCL.

FIG. 2B shows the relative BODIPY-starch hydrolysis activity versus relative net charge change for an AmyS-S242Q CCL.

FIG. 3A shows the relative shake tube expression versus relative net charge change for an AmyS-S242Q CCL.

FIG. 3B shows the rice starch microswatch cleaning activity versus relative net charge change for an AmyS-S242Q CCL.

## GENERAL DESCRIPTION OF THE INVENTION

The present invention provides methods for engineering enzymes to optimize their performance under certain environmental conditions of interest. In some embodiments, the present invention provides methods for engineering enzymes to optimize their catalytic activity under particular environmental conditions. In some preferred embodiments, the present invention provides methods for engineering enzymes to optimize their catalytic activity and/or stability under adverse environmental conditions. In some preferred embodiments, the present invention provides methods for engineering enzymes to optimize their stability, particularly under adverse environmental conditions. In some preferred embodiments, the present invention provides methods for altering the net surface charge and/or surface charge distribution of enzymes (e.g., metalloproteases) to obtain enzyme variants that demonstrate improved performance and/or stability in detergent formulations as compared to the starting or parent enzyme.

The protease subtilisin is a major enzyme used in laundry detergents and perhaps the most widely used enzyme in the world. It has been noted that surface electrostatic effects could modulate the catalytic activity of subtilisin (See e.g., Russell and Fersht, Nature 328:496-500 [1987]). More recently, mutations that involved changing the net charge of subtilisin were observed to have a dramatic effect on wash performance in detergents (See e.g., EP Patent No. 0 479 870 BI). This beneficial effect was believed to be a result of shifting the pI (isoelectric point) of subtilisin toward the pH of the wash liquor. However, later work demonstrated that this conclusion is not always applicable (See e.g., US Patent No. 6,673,590 B1). As indicated in this Patent, the effect of charge mutations in subtilisin depend dramatically on detergent concentrations, with mutations lowering the pI of the parent subtilisin providing an enzyme that is more effective at low detergent concentration and mutations raising the pI providing an enzyme that is more effective at high detergent concentration. This is of great utility because detergent concentration in the wash liquors varies greatly across the globe. Thus, it has become apparent to those of skill in the art that there is an optimal pI for wash performance of subtilisin, which depends on the pH and detergent concentration in the wash liquor. Further efforts to improve the activity of subtilisin in laundry detergents have been described (See, US Pat. Publication No. 2005/0221461). Surprisingly, subtilisin variants having the same net electrostatic charge as the parent subtilisin were found to have increased wash performance under both high and low detergent concentration wash conditions.

Unless otherwise indicated, the practice of the present invention involves conventional techniques commonly used in protein engineering, molecular biology, microbiology, and recombinant DNA, which are within the skill of the art. Such techniques are known to those of skill in the art and are described in numerous texts and reference works well-known to those skilled in the art.

Unless defined otherwise herein, all technical and scientific terms used herein have the same meaning as commonly understood by one of ordinary skill in the art to which this invention pertains. Although any methods and materials similar or equivalent to those described herein find use in the practice of the present invention, some of the preferred methods and materials are described herein. Accordingly, the terms defined immediately below are more fully described by reference to the Specification as a whole.

Also, as used herein, the singular "a," "an," and "the" include the plural reference unless the context clearly indicates otherwise. Numeric ranges are inclusive of the numbers defining the range. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Furthermore, the headings provided herein are not limitations of the various aspects or embodiments of the invention, which can be had by reference to the specification as a whole. Accordingly, the terms defined immediately below are more fully defined by reference to the specification as a whole. Nonetheless, in order to facilitate understanding of the invention, a number of terms are defined below.

**Definitions**

As used herein, the terms "protease," and "proteolytic activity" refer to a protein or peptide exhibiting the ability to hydrolyze peptides or substrates having peptide linkages. Many well known procedures exist for measuring proteolytic activity *(See e.g.,* Kalisz, "Microbial Proteinases," In: Fiechter (ed.), Advances in Biochemical Engineering/Biotechnology, [1988]). For example, proteolytic activity may be ascertained by comparative assays, which analyze the respective protease's ability to hydrolyze a commercial substrate. Exemplary substrates useful in such analysis of protease or proteolytic activity, include, but are not limited to di-methyl casein (Sigma C-9801), bovine collagen (Sigma C-9879), bovine elastin (Sigma E-1625), and bovine keratin (ICN Biomedical 942111). Colorimetric assays utilizing these substrates are well known in the art (*See e.g.,* WO 99/34011; and U.S. Patent No. 6,376,450). The pNA assay *(See e.g.,* Del Mar et al., Anal Biochem, 99:316-320 [1979]) also finds use in determining the active enzyme concentration for fractions collected during gradient elution. This assay measures the rate at which *p*-nitroaniline is released as the enzyme hydrolyzes the soluble synthetic substrate, succinyl-alanine-alanine-proline-phenylalanine-*p*-nitroanilide (sAAPF-*p*NA). the rate of production of yellow color from the hydrolysis reaction is measured at 410 nm on a spectrophotometer and is proportional to the active enzyme concentration. In addition, absorbance measurements at 280 nm can be used to determine the total protein concentration. The active enzyme/total-protein ratio gives the enzyme purity.

As used herein, the terms "ASP protease," "Asp protease" and "Asp," refer to the serine Patent No. 8,535,927 proteases described herein and described in U.S. Patent No. 8,535,927. In some preferred embodiments, the Asp protease is the protease designed herein as 69B4 protease obtained from *Cellulomanas* strain 69B4. Thus, in preferred embodiments, the term "69B4 protease" refers to a naturally occurring mature protease derived from *Cellulomonas* strain 69B4 (DSM 16035).

The term *"Cellulomanas* protease homologues" refers to naturally occurring proteases having substantially identical amino acid sequences to the mature protease derived from *Cellulamonas* strain 69B4 or polynucleotide sequences which encode for such naturally occurring, proteases, and which proteases retain the functional characteristics of a serine protease encoded by such nucleic acids. In some embodiments, these protease homologues are referred to as "cellulomonadins."

As used herein, the terms "ASP variant," "ASP protease variant," and "69B protease variant" are used in reference to proteases that are similar to the wild-type ASP, particularly in their function, but have mutations in their amino acid sequence that make them different in sequence from the wild-type protease.

As used herein, *"Cellulomonas* ssp." refers to all of the species within the genus *"Cellulomonas,"* which are Gram-positive bacteria classified as members of the Family *Cellulomonadaceae,* Suborder *Micrococcineae,* Order *Ac-*

*tinomycetales,* Class *Actinobacteria.* It is recognized that the genus *Cellulomonas* continues to undergo taxonomical reorganization. Thus, it is intended that the genus include species that have been reclassified.

As used herein, *"Streptomyces* ssp." refers to all of the species within the genus *"Streptomyces,"* which are Gram-positive bacteria classified as members of the Family *Streptomycetaceae,* Suborder *Streptomycineae,* Order *Actinomycetales,* class *Actinobacteria.* It is recognized that the genus *Streptomyces* continues to undergo taxonomical reorganization. Thus, it is intended that the genus include species that have been reclassified

As used herein, "the genus *Bacillus"* includes all species within the genus *"Bacillus,"* as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus."* The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

The terms "polynucleotide" and "nucleic acid", used interchangeably herein, refer to a polymeric form of nucleotides of any length, either ribonucleotides or deoxyribonucleotides. These terms include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: genes, gene fragments, chromosomal fragments, ESTs, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In some embodiments, polynucleotides comprise modified nucleotides, such as methylated nucleotides and nucleotide analogs, uracil, other sugars and linking groups such as fluororibose and thioate, and nucleotide branches. In alternative embodiments, the sequence of nucleotides is interrupted by non-nucleotide components.

As used herein, the terms "DNA construct" and "transforming DNA" are used interchangeably to refer to DNA used to introduce sequences into a host cell or organism. The DNA may be generated *in vitro* by PCR or any other suitable technique(s) known to those in the art. In particularly preferred embodiments, the DNA construct comprises a sequence of interest (*e.g.,* as an incoming sequence). In some embodiments, the sequence is operably linked to additional elements such as control elements (*e.g.,* promoters, etc.). The DNA construct may further comprise a selectable marker. It may further comprise an incoming sequence flanked by homology boxes. In a further embodiment, the transforming DNA comprises other non-homologous sequences, added to the ends (*e.g.,* stuffer sequences or flanks). In some embodiments, the ends of the incoming sequence are closed such that the transforming DNA forms a closed circle. The transforming sequences may be wild-type, mutant or modified. In some embodiments, the DNA construct comprises sequences homologous to the host cell chromosome. In other embodiments, the DNA construct comprises non-homologous sequences. Once the DNA construct is assembled *in vitro* it may be used to: 1) insert heterologous sequences into a desired target sequence of a host cell; and/or 2) mutagenize a region of the host cell chromosome (*i.e.,* replace an endogenous sequence with a heterologous sequence), and/or 3) delete target genes; and/or introduce a replicating plasmid into the host.

As used herein, the terms "expression cassette" and "expression vector" refer to nucleic acid constructs generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In preferred embodiments, expression vectors have the ability to incorporate and express heterologous DNA fragments in a host cell. Many prokaryotic and eukaryotic expression vectors are commercially available. Selection of appropriate expression vectors is within the knowledge of those of skill in the art. The term "expression cassette" is used interchangeably herein with "DNA construct," and their grammatical equivalents. Selection of appropriate expression vectors is within the knowledge of those of skill in the art.

As used herein, the term "vector" refers to a polynucleotide construct designed to introduce nucleic acids into one or more cell types. Vectors include cloning vectors, expression vectors, shuttle vectors, plasmids, cassettes and the like. In some embodiments, the polynucleotide construct comprises a DNA sequence encoding the protease (*e.g.,* precursor or mature protease) that is operably linked to a suitable prosequence (*e.g.,* secretory, etc.) capable of effecting the expression of the DNA in a suitable host.

As used herein, the term "plasmid" refers to a circular double-stranded (ds) DNA construct used as a cloning vector, and which forms an extrachromosomal self-replicating genetic element in some eukaryotes or prokaryotes, or integrates into the host chromosome.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, conjugation, and transduction *(See e.g.,* Ferrari et al., "Genetics. " in Hardwood et al, (eds.), Bacillus, Plenum Publishing Corp., pages 57-72 [1989]).

As used herein, the terms "transformed" and "stably transformed" refer to a cell that has a non-native (heterologous) polynucleotide sequence integrated into its genome or as an episomal plasmid that is maintained for at least two generations.

As used herein, the term "selectable marker-encoding nucleotide sequence" refers to a nucleotide sequence, which is capable of expression in host cells and where expression of the selectable marker confers to cells containing the expressed gene the ability to grow in the presence of a corresponding selective agent or lack of an essential nutrient.

As used herein, the terms "selectable marker" and "selective marker" refer to a nucleic acid (*e.g.*, a gene) capable of expression in host cell which allows for ease of selection of those hosts containing the vector. Examples of such selectable markers include but are not limited to antimicrobials. Thus, the term "selectable marker" refers to genes that provide an indication that a host cell has taken up an incoming DNA of interest or some other reaction has occurred. Typically, selectable markers are genes that confer antimicrobial resistance or a metabolic advantage on the host cell to allow cells containing the exogenous DNA to be distinguished from cells that have not received any exogenous sequence during the transformation. A "residing selectable marker" is one that is located on the chromosome of the microorganism to be transformed. A residing selectable marker encodes a gene that is different from the selectable marker on the transforming DNA construct. Selective markers are well known to those of skill in the art. As indicated above, preferably the marker is an antimicrobial resistant marker (*e.g.*, amp$^R$; phleo$^R$; spec$^R$; kan$^R$; ery$^R$; tet$^R$; cmp$^R$; and neo$^R$ *(See e.g.,* Guerot-Fleury, Gene, 167:335-337 [1995]; Palmeros et al., Gene 247:255-264 [2000]; and Trieu-Cuot et al., Gene, 23:331-341 [1983]). Other markers useful in accordance with the invention include, but are not limited to auxotrophic markers, such as tryptophan; and detection markers, such as β- galactosidase.

As used herein, the term "promoter" refers to a nucleic acid sequence that functions to direct transcription of a downstream gene. In preferred embodiments, the promoter is appropriate to the host cell in which the target gene is being expressed. The promoter, together with other transcriptional and translational regulatory nucleic acid sequences (also termed "control sequences") is necessary to express a given gene. In general, the transcriptional and translational regulatory sequences include, but are not limited to, promoter sequences, ribosomal binding sites, transcriptional start and stop sequences, translational start and stop sequences, and enhancer or activator sequences.

A nucleic acid is "operably linked" when it is placed into a functional relationship with another nucleic acid sequence. For example, DNA encoding a secretory leader *(i.e.,* a signal peptide), is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein the term "gene" refers to a polynucleotide (*e.g.*, a DNA segment) that encodes a polypeptide and includes regions preceding and following the coding regions as well as intervening sequences (introns) between individual coding segments (exons).

As used herein, "homologous genes" refers to a pair of genes from different, but usually related species, which correspond to each other and which are identical or very similar to each other. The term encompasses genes that are separated by speciation *(i.e.,* the development of new species) (*e.g.*, orthologous genes), as well as genes that have been separated by genetic duplication (*e.g.*, paralogous genes).

As used herein, "ortholog" and "orthologous genes" refer to genes in different species that have evolved from a common ancestral gene *(i.e.,* a homologous gene) by speciation. Typically, orthologs retain the same function during the course of evolution. Identification of orthologs finds use in the reliable prediction of gene function in newly sequenced genomes.

As used herein, "paralog" and "paralogous genes" refer to genes that are related by duplication within a genome. While orthologs retain the same function through the course of evolution, paralogs evolve new functions, even though some functions are often related to the original one. Examples of paralogous genes include, but are not limited to genes encoding trypsin, chymotrypsin, elastase, and thrombin, which are all serine proteinases and occur together within the same species.

As used herein, "homology" refers to sequence similarity or identity, with identity being preferred. This homology is determined using standard techniques known in the art *(See e.g.,* Smith and Waterman, Adv. Appl. Math., 2:482 [1981]; Needleman and Wunsch, J. Mol. Biol., 48:443 [1970]; Pearson and Lipman, Proc. Natl. Acad .Sci. USA, 85:2444 [1988]; programs such as GAP, BESTFIT, FASTA, and TFASTA in the Wisconsin Genetics Software Package, Genetics Computer Group, Madison, WI; and Devereux et al., Nucl. Acid Res., 12:387-395 [1984]).

As used herein, an "analogous sequence" is one wherein the function of the gene is essentially the same as the gene based on a parent gene *(e.g.,* the *Cellulomonas* strain 69B4 protease). Additionally, analogous genes include at least about 45%, about 50%, about 55%, about 60%, about 65%, about 70%, about 75%, about 80%, about 85%, about 90%, about 95%, about 97%, about 98%, about 99% or about 100% sequence identity with the sequence of the parent gene. Alternately, analogous sequences have an alignment of between 70 to 100% of the genes found in the parent gene *(e.g., Cellulomonas* strain 69B4 protease) region and/or have at least between 5-10 genes found in the region aligned with the genes in the chromosome containing the parent gene *(e.g.,* the *Cellulomonas* strain 69B4 chromosome). In additional embodiments more than one of the above properties applies to the sequence. Analogous sequences are determined by known methods of sequence alignment. A commonly used alignment method is BLAST, although as indicated above and below, there are other methods that also find use in aligning sequences.

One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters include a default gap weight of 3.00, a default gap length weight of 0.10, and weighted end gaps.

Another example of a useful algorithm is the BLAST algorithm, described by Altschul *et al.,* (Altschul et al., J. Mol. Biol., 215:403-410 [1990]; and Karlin et al., Proc. Natl. Acad. Sci., USA, 90:5873-5787 [1993]). A particularly useful BLAST program is the WU-BLAST-2 program *(See,* Altschul et al., Meth. Enzymol., 266:460-480 [1996]). WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

Thus, "percent (%) nucleic acid sequence identity" is defined as the percentage of nucleotide residues in a candidate sequence that are identical to the nucleotide residues of the starting sequence *(i.e.,* the sequence of interest). A preferred method utilizes the BLASTN module of WU-BLAST-2 set to the default parameters, with overlap span and overlap fraction set to 1 and 0.125, respectively.

As used herein, the term "hybridization" refers to the process by which a strand of nucleic acid joins with a complementary strand through base pairing, as known in the art.

A nucleic acid sequence is considered to be "selectively hybridizable" to a reference nucleic acid sequence if the two sequences specifically hybridize to one another under moderate to high stringency hybridization and wash conditions. Hybridization conditions are based on the melting temperature (Tm) of the nucleic acid binding complex or probe. For example, "maximum stringency" typically occurs at about Tm-5°C (5° below the Tm of the probe); "high stringency" at about 5-10°C below the Tm; "intermediate stringency" at about 10-20°C below the Tm of the probe; and "low stringency" at about 20-25°C below the Tm. Functionally, maximum stringency conditions may be used to identify sequences having strict identity or near-strict identity with the hybridization probe; while an intermediate or low stringency hybridization can be used to identify or detect polynucleotide sequence homologs.

Moderate and high stringency hybridization conditions are well known in the art. An example of high stringency conditions includes hybridization at about 42°C in 50% formamide, 5X SSC, 5X Denhardt's solution, 0.5% SDS and 100 μg/ml denatured carrier DNA followed by washing two times in 2X SSC and 0.5% SDS at room temperature and two additional times in 0.1X SSC and 0.5% SDS at 42°C. An example of moderate stringent conditions include an overnight incubation at 37°C in a solution comprising 20% formamide, 5 x SSC (150mM NaCl, 15 mM trisodium citrate), 50 mM sodium phosphate (pH 7.6), 5 x Denhardt's solution, 10% dextran sulfate and 20 mg/ml denatured sheared salmon sperm DNA, followed by washing the filters in 1x SSC at about 37 - 50°C. Those of skill in the art know how to adjust the temperature, ionic strength, etc. as necessary to accommodate factors such as probe length and the like.

As used herein, "recombinant" includes reference to a cell or vector, that has been modified by the introduction of a heterologous nucleic acid sequence or that the cell is derived from a cell so modified. Thus, for example, recombinant cells express genes that are not found in identical form within the native (non-recombinant) form of the cell or express native genes that are otherwise abnormally expressed, under expressed or not expressed at all as a result of deliberate human intervention. "Recombination," "recombining," and generating a "recombined" nucleic acid are generally the assembly of two or more nucleic acid fragments wherein the assembly gives rise to a chimeric gene.

In a preferred embodiment, mutant DNA sequences are generated with site saturation mutagenesis in at least one codon. In another preferred embodiment, site saturation mutagenesis is performed for two or more codons. In a further embodiment, mutant DNA sequences have more than 50%, more than 55%, more than 60%, more than 65%, more than 70%, more than 75%, more than 80%, more than 85%, more than 90%, more than 95%, or more than 98% homology with the wild-type sequence. In alternative embodiments, mutant DNA is generated *in vivo* using any known mutagenic procedure such as, for example, radiation, nitrosoguanidine and the like. The desired DNA sequence is then isolated and used in the methods provided herein.

As used herein, the term "target sequence" refers to a DNA sequence in the host cell that encodes the sequence where it is desired for the incoming sequence to be inserted into the host cell genome. In some embodiments, the target sequence encodes a functional wild-type gene or operon, while in other embodiments the target sequence encodes a functional mutant gene or operon, or a non-functional gene or operon.

As used herein, a "flanking sequence" refers to any sequence that is either upstream or downstream of the sequence being discussed (e.g., for genes A-B-C, gene B is flanked by the A and C gene sequences). In a preferred embodiment, the incoming sequence is flanked by a homology box on each side. In another embodiment, the incoming sequence and the homology boxes comprise a unit that is flanked by stuffer sequence on each side. In some embodiments, a flanking sequence is present on only a single side (either 3' or 5'), but in preferred embodiments, it is on each side of the sequence being flanked. In some embodiments, a flanking sequence is present on only a single side (either 3' or 5'), while in preferred embodiments, it is present on each side of the sequence being flanked.

As used herein, the term "stuffer sequence" refers to any extra DNA that flanks homology boxes (typically vector sequences). However, the term encompasses any non-homologous DNA sequence. Not to be limited by any theory, a stuffer sequence provides a noncritical target for a cell to initiate DNA uptake.

As used herein, the terms "amplification" and "gene amplification" refer to a process by which specific DNA sequences are disproportionately replicated such that the amplified gene becomes present in a higher copy number than was initially present in the genome. In some embodiments, selection of cells by growth in the presence of a drug (*e.g.,* an inhibitor of an inhibitable enzyme) results in the amplification of either the endogenous gene encoding the gene product required for growth in the presence of the drug or by amplification of exogenous (*i.e.,* input) sequences encoding this gene product, or both.

"Amplification" is a special case of nucleic acid replication involving template specificity. It is to be contrasted with non-specific template replication (*i.e.,* replication that is template-dependent but not dependent on a specific template). Template specificity is here distinguished from fidelity of replication (*i.e.,* synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

As used herein, the term "co-amplification" refers to the introduction into a single cell of an amplifiable marker in conjunction with other gene sequences (*i.e.,* comprising one or more non-selectable genes such as those contained within an expression vector) and the application of appropriate selective pressure such that the cell amplifies both the amplifiable marker and the other, non-selectable gene sequences. The amplifiable marker may be physically linked to the other gene sequences or alternatively two separate pieces of DNA, one containing the amplifiable marker and the other containing the non-selectable marker, may be introduced into the same cell.

As used herein, the terms "amplifiable marker," "amplifiable gene," and "amplification vector" refer to a gene or a vector encoding a gene, which permits the amplification of that gene under appropriate growth conditions.

"Template specificity" is achieved in most amplification techniques by the choice of enzyme. Amplification enzymes are enzymes that, under conditions they are used, will process only specific sequences of nucleic acid in a heterogeneous mixture of nucleic acid. For example, in the case of Qβ replicase, MDV-1 RNA is the specific template for the replicase (*See e.g.,* Kacian et al., Proc. Natl. Acad. Sci. USA 69:3038 [1972]) and other nucleic acids are not replicated by this amplification enzyme. Similarly, in the case of T7 RNA polymerase, this amplification enzyme has a stringent specificity for its own promoters (*See,* Chamberlin et al., Nature 228:227 [1970]). In the case of T4 DNA ligase, the enzyme will not ligate the two oligonucleotides or polynucleotides, where there is a mismatch between the oligonucleotide or polynucleotide substrate and the template at the ligation junction (*See,* Wu and Wallace, Genomics 4:560 [1989]). Finally, *Taq* and *Pfu* polymerases, by virtue of their ability to function at high temperature, are found to display high specificity for the sequences bounded and thus defined by the primers; the high temperature results in thermodynamic conditions that favor primer hybridization with the target sequences and not hybridization with non-target sequences.

As used herein, the term "amplifiable nucleic acid" refers to nucleic acids which may be amplified by any amplification method. It is contemplated that "amplifiable nucleic acid" will usually comprise "sample template."

As used herein, the term "sample template" refers to nucleic acid originating from a sample which is analyzed for the presence of "target" (defined below). In contrast, "background template" is used in reference to nucleic acid other than sample template, which may or may not be present in a sample. Background template is most often inadvertent. It may be the result of carryover, or it may be due to the presence of nucleic acid contaminants sought to be purified away from the sample. For example, nucleic acids from organisms other than those to be detected may be present as background in a test sample.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, (i.e., in the presence of nucleotides and an inducing agent such as DNA polymerase and at a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and the use of the method.

As used herein, the term "probe" refers to an oligonucleotide (i.e., a sequence of nucleotides), whether occurring naturally as in a purified restriction digest or produced synthetically, recombinantly or by PCR amplification, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A "segment" is defined as a region of nucleic acid within the target sequence.

As used herein, the term "polymerase chain reaction" ("PCR") refers to the methods of U.S. Patent Nos. 4,683,195 4,683,202, and 4,965,188, which include methods for increasing the concentration of a segment of a target sequence in a mixture of genomic DNA without cloning or purification, as known to those of skill in the art. Because the desired amplified segments of the target sequence become the predominant sequences (in terms of concentration) in the mixture, they are said to be "PCR amplified".

As used herein, the term "amplification reagents" refers to those reagents (deoxyribonucleotide triphosphates, buffer, etc.), needed for amplification except for primers, nucleic acid template and the amplification enzyme. Typically, amplification reagents along with other reaction components are placed and contained in a reaction vessel (test tube, microwell, etc.).

With PCR, it is possible to amplify a single copy of a specific target sequence in genomic DNA to a level detectable by several different methodologies (e.g., hybridization with a labeled probe; incorporation of biotinylated primers followed by avidin-enzyme conjugate detection; incorporation of $^{32}$P-labeled deoxynucleotide triphosphates, such as dCTP or dATP, into the amplified segment). In addition to genomic DNA, any oligonucleotide or polynucleotide sequence can be amplified with the appropriate set of primer molecules. In particular, the amplified segments created by the PCR process itself are, themselves, efficient templates for subsequent PCR amplifications.

As used herein, the terms "PCR product," "PCR fragment," and "amplification product" refer to the resultant mixture of compounds after two or more cycles of the PCR steps of denaturation, annealing and extension are complete. These terms encompass the case where there has been amplification of one or more segments of one or more target sequences.

As used herein, the term "RT-PCR" refers to the replication and amplification of RNA sequences. In this method, reverse transcription is coupled to PCR, most often using a one enzyme procedure in which a thermostable polymerase is employed, as described in U.S. Patent No. 5,322,770. In RT-PCR, the RNA template is converted to cDNA due to the reverse transcriptase activity of the polymerase, and then amplified using the polymerizing activity of the polymerase (i.e., as in other PCR methods).

As used herein, the terms "restriction endonucleases" and "restriction enzymes" refer to bacterial enzymes, each of which cut double-stranded DNA at or near a specific nucleotide sequence.

A "restriction site" refers to a nucleotide sequence recognized and cleaved by a given restriction endonuclease and is frequently the site for insertion of DNA fragments. In certain embodiments of the invention restriction sites are engineered into the selective marker and into 5' and 3' ends of the DNA construct.

As used herein, the term "chromosomal integration" refers to the process whereby an incoming sequence is introduced into the chromosome of a host cell. The homologous regions of the transforming DNA align with homologous regions of the chromosome. Subsequently, the sequence between the homology boxes is replaced by the incoming sequence in a double crossover (i.e., homologous recombination). In some embodiments of the present invention, homologous sections of an inactivating chromosomal segment of a DNA construct align with the flanking homologous regions of the indigenous chromosomal region of the *Bacillus* chromosome. Subsequently, the indigenous chromosomal region is deleted by the DNA construct in a double crossover (i.e., homologous recombination).

"Homologous recombination" means the exchange of DNA fragments between two DNA molecules or paired chromosomes at the site of identical or nearly identical nucleotide sequences. In a preferred embodiment, chromosomal integration is homologous recombination.

"Homologous sequences" as used herein means a nucleic acid or polypeptide sequence having 100%, 99%, 98%, 97%, 96%, 95%, 94%, 93%, 92%, 91 %, 90%, 88%, 85%, 80%, 75%, or 70% sequence identity to another nucleic acid or polypeptide sequence when optimally aligned for comparison. In some embodiments, homologous sequences have between 85% and 100% sequence identity, while in other embodiments there is between 90% and 100% sequence identity, and in more preferred embodiments, there is 95% and 100% sequence identity.

As used herein "amino acid" refers to peptide or protein sequences or portions thereof. The terms "protein," "peptide," and "polypeptide" are used interchangeably.

As used herein, "protein of interest" and "polypeptide of interest" refer to a protein/polypeptide that is desired and/or being assessed. In some embodiments, the "protein of interest" is a "parent protein" *(i.e.,* the starting protein). In some embodiments, the parent protein is a wild-type enzyme that is used as a starting point for protein engineering/design. In some embodiments, the protein of interest is expressed intracellularly, while in other embodiments, it is a secreted polypeptide. In particularly preferred embodiments, these enzymes include the serine proteases and metalloproteases described herein. In some embodiments, the protein of interest is a secreted polypeptide fused to a signal peptide *(i.e.,* an amino-terminal extension on a protein to be secreted). Nearly all secreted proteins use an amino-terminal protein extension, which plays a crucial role in the targeting to and translocation of precursor proteins across the membrane. This extension is proteolytically removed by a signal peptidase during or immediately following membrane transfer.

As used herein, the term "heterologous protein" refers to a protein or polypeptide that does not naturally occur in the host cell. Examples of heterologous proteins include enzymes such as hydrolases including proteases. In some embodiments, the gene encoding the proteins are naturally occurring genes, while in other embodiments, mutated and/or synthetic genes are used.

As used herein, "homologous protein" refers to a protein or polypeptide native or naturally occurring in a cell. In preferred embodiments, the cell is a Gram-positive cell, while in particularly preferred embodiments, the cell is a *Bacillus* host cell. In alternative embodiments, the homologous protein is a native protein produced by other organisms, including but not limited to *E. coli, Cellulomonas, Bacillus, Streptomyces, Trichoderma,* and *Aspergillus.*

As used herein, an "operon region" comprises a group of contiguous genes that are transcribed as a single transcription unit from a common promoter, and are thereby subject to co-regulation. In some embodiments, the operon includes a regulator gene. In most preferred embodiments, operons that are highly expressed as measured by RNA levels, but have an unknown or unnecessary function are used.

As used herein, an "antimicrobial region" is a region containing at least one gene that encodes an antimicrobial protein.

A polynucleotide is said to "encode" an RNA or a polypeptide if, in its native state or when manipulated by methods known to those of skill in the art, it can be transcribed and/or translated to produce the RNA, the polypeptide or a fragment thereof. The anti-sense strand of such a nucleic acid is also said to encode the sequences.

As is known in the art, a DNA can be transcribed by an RNA polymerase to produce RNA, but an RNA can be reverse transcribed by reverse transcriptase to produce a DNA. Thus a DNA can encode a RNA and vice versa.

The term "regulatory segment" or "regulatory sequence" or "expression control sequence" refers to a polynucleotide sequence of DNA that is operatively linked with a polynucleotide sequence of DNA that encodes the amino acid sequence of a polypeptide chain to effect the expression of the encoded amino acid sequence. The regulatory sequence can inhibit, repress, or promote the expression of the operably linked polynucleotide sequence encoding the amino acid.

"Host strain" or "host cell" refers to a suitable host for an expression vector comprising DNA according to the present invention.

An enzyme is "overexpressed" in a host cell if the enzyme is expressed in the cell at a higher level that the level at which it is expressed in a corresponding wild-type cell.

The terms "protein" and "polypeptide" are used interchangeability herein. The 3-letter code for amino acids as defined in conformity with the IUPAC-IUB Joint Commission on Biochemical Nomenclature (JCBN) is used through out this disclosure. It is also understood that a polypeptide may be coded for by more than one nucleotide sequence due to the degeneracy of the genetic code.

A "prosequence" is an amino acid sequence between the signal sequence and mature protease that is necessary for the secretion of the protease. Cleavage of the pro sequence will result in a mature active protease.

The term "signal sequence" or "signal peptide" refers to any sequence of nucleotides and/or amino acids that participate in the secretion of the mature or precursor forms of the protein. This definition of signal sequence is a functional one, meant to include all those amino acid sequences encoded by the N-terminal portion of the protein gene, which participate in the effectuation of the secretion of protein. They are often, but not universally, bound to the N-terminal portion of a protein or to the N-terminal portion of a precursor protein. The signal sequence may be endogenous or exogenous. The signal sequence may be that normally associated with the protein (*e.g.*, protease), or may be from a gene encoding another secreted protein. One exemplary exogenous signal sequence comprises the first seven amino acid residues of the signal sequence from *B. sublilis* subtilisin fused to the remainder of the signal sequence of the subtilisin from *B. lentus* (ATCC 21536).

The term "hybrid signal sequence" refers to signal sequences in which part of sequence is obtained from the expression host fused to the signal sequence of the gene to be expressed. In some embodiments, synthetic sequences are utilized.

The term "substantially the same signal activity" refers to the signal activity, as indicated by substantially the same secretion of the protease into the fermentation medium, for example a fermentation medium protease level being at least 50%, at least 60%, at least 70%, at least 80%, at least 90%, at least 95%, at least 98% of the secreted protease levels in the fermentation medium as provided by a signal sequence.

The term "mature" form of a protein or peptide refers to the final functional form of the protein or peptide. To exemplify, a mature form of the NprE protease least includes the amino acid sequence of SEQ ID NO:3.

The term "precursor" form of a protein or peptide refers to a mature form of the protein having a prosequence operably linked to the amino or carbonyl terminus of the protein. The precursor may also have a "signal" sequence operably linked, to the amino terminus of the prosequence. The precursor may also have additional polynucleotides that are involved in post-translational activity (*e.g.*, polynucleotides cleaved therefrom to leave the mature form of a protein or peptide).

"Naturally occurring enzyme" and "naturally occurring protein" refer to an enzyme or protein having the unmodified amino acid sequence identical to that found in nature. Naturally occurring enzymes include native enzymes, those enzymes naturally expressed or found in the particular microorganism.

The terms "derived from" and "obtained from" refer to not only an enzyme (*e.g.*, protease) produced or producible by a strain of the organism in question, but also an enzyme encoded by a DNA sequence isolated from such strain and produced in a host organism containing such DNA sequence. Additionally, the term refers to a enzyme that is encoded by a DNA sequence of synthetic and/or cDNA origin and which has the identifying characteristics of the enzyme in question.

A "derivative" within the scope of this definition generally retains the characteristic proteolytic activity observed in the wild-type, native or parent form to the extent that the derivative is useful for similar purposes as the wild-type, native or parent form. Functional enzyme derivatives encompass naturally occurring, synthetically or recombinantly produced peptides or peptide fragments having the general characteristics of the parent enzyme.

The term "functional derivative" refers to a derivative of a nucleic acid having the functional characteristics of a nucleic acid encoding an enzyme. Functional derivatives of a nucleic acid, which encode enzymes provided herein encompass naturally occurring, synthetically or recombinantly produced nucleic acids or fragments. Wild type nucleic acid encoding enzymes according to the present invention include naturally occurring alleles and homologues based on the degeneracy of the genetic code known in the art.

The term "identical" in the context of two nucleic acids or polypeptide sequences refers to the residues in the two sequences that are the same when aligned for maximum correspondence, as measured using one of the following sequence comparison or analysis algorithms.

The term "optimal alignment" refers to the alignment giving the highest percent identity score.

"Percent sequence identity," "percent amino acid sequence identity," "percent gene sequence identity," and/or "percent nucleic acid/polynucloetide sequence identity," with respect to two amino acid, polynucleotide and/or gene sequences (as appropriate), refer to the percentage of residues that are identical in the two sequences when the sequences are optimally aligned. Thus, 80% amino acid sequence identity means that 80% of the amino acids in two optimally aligned polypeptide sequences are identical.

The phrase "substantially identical" in the context of two nucleic acids or polypeptides thus refers to a polynucleotide or polypeptide that comprising at least 70% sequence identity, preferably at least 75%, preferably at least 80%, preferably at least 85%, preferably at least 90%, preferably at least 95%, preferably at least 97% , preferably at least 98% and preferably at least 99% sequence identity as compared to a reference sequence using the programs or algorithms (e.g., BLAST, ALIGN, CLUSTAL) using standard parameters. One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution. Another indication that two nucleic acid sequences are substantially identical is that the two molecules hybridize to each other under stringent conditions (e.g., within a range of medium to high stringency).

The term "isolated" or "purified" refers to a material that is removed from its original environment (*e.g.*, the natural environment if it is naturally occurring). For example, the material is said to be "purified" when it is present in a particular composition in a higher or lower concentration than exists in a naturally occurring or wild type organism or in combination with components not normally present upon expression from a naturally occurring or wild type organism. For example, a naturally-occurring polynucleotide or polypeptide present in a living animal is not isolated, but the same polynucleotide or polypeptide, separated from some or all of the coexisting materials in the natural system, is isolated. In some embodiments, such polynucleotides are part of a vector, and/or such polynucleotides or polypeptides are part of a composition, and still be isolated in that such vector or composition is not part of its natural environment. In some preferred embodiments, a nucleic acid or protein is said to be purified, for example, if it gives rise to essentially one band in an electrophoretic gel or blot.

The term "isolated," when used in reference to a DNA sequence, refers to a DNA sequence that has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art *(See e.g.,* Dynan and Tijan, Nature 316:774-78, 1985). The term "an isolated DNA sequence" is alternatively referred to as "a cloned DNA sequence".

The term "isolated," when used in reference to a protein, refers to a protein that is found in a condition other than its native environment. In a preferred form, the isolated protein is substantially free of other proteins, particularly other homologous proteins. An isolated protein is more than 10% pure, preferably more than 20% pure, and even more preferably more than 30% pure, as determined by SDS-PAGE. As used herein, protein in a highly purified form is more than 40% pure, more than 60% pure, more than 80% pure, more than 90% pure, more than 95% pure, more than 97% pure, and even more than 99% pure), as determined by SDS-PAGE.

As used herein, the term, "combinatorial mutagenesis" refers to methods in which libraries of variants of a starting sequence are generated. In these libraries, the variants contain one or several mutations chosen from a predefined set of mutations. In addition, the methods provide means to introduce random mutations, which were not members of the predefined set of mutations. In some embodiments, the methods include those set forth in U.S. Patent No. 6,582,914. In alternative embodiments, combinatorial mutagenesis methods encompass commercially available kits (*e.g.,* QUIKCHANGE® Multisite, Stratagene, La Jolla, CA).

As used herein, the term "variant" refers to a protein that has been derived from a precursor protein (*e.g.,* "parent" protein) by addition, substitution, or deletion of one or more amino acids. In some embodiments, the variant comprises at least one modification that comprises a change in charge, as compared to the precursor protein. In some preferred embodiments, the precursor protein is a parent protein that is a wild-type protein.

As used herein, the term "library of mutants" refers to a population of cells which are identical in most of their genome but include different homologues of one or more genes. Such libraries can be used, for example, to identify genes or operons with improved traits.

As used herein, the term "starting gene" refers to a gene of interest that encodes a protein of interest that is to be improved and/or changed using the present invention.

As used herein, the terms "multiple sequence alignment" and "MSA" refer to the sequences of multiple homologs of a starting gene that are aligned using an algorithm (*e.g.*, Clustal W).

As used herein, the terms "consensus sequence" and "canonical sequence" refer to an archetypical amino acid sequence against which all variants of a particular protein or sequence of interest are compared, The terms also refer to a sequence that sets forth the nucleotides that are most often present in a DNA sequence of interest. For each position of a gene, the consensus sequence gives the amino acid that is most abundant in that position in the MSA.

As used herein, the term "consensus mutation" refers to a difference in the sequence of a starting gene and a consensus sequence. Consensus mutations are identified by comparing the sequences of the starting gene and the consensus sequence obtained from a MSA. In some embodiments, consensus mutations are introduced into the starting gene such that it becomes more similar to the consensus sequence. Consensus mutations also include amino acid changes that change an amino acid in a starting gene to an amino acid that is more frequently found in an MSA at that position relative to the frequency of that amino acid in the starting gene. Thus, the term consensus mutation comprises all single amino acid changes that replace an amino acid of the starting gene with an amino acid that is more abundant than the amino acid in the MSA.

As used herein, the term "initial hit" refers to a variant that was identified by screening a combinatorial consensus mutagenesis library. In preferred embodiments, initial hits have improved performance characteristics, as compared to the starting gene.

As used herein, the term "improved hit" refers to a variant that was identified by screening an enhanced combinatorial consensus mutagenesis library.

As used herein, the terms "improving mutation" and "performance-enhancing mutation" refer to a mutation that leads to improved performance when it is introduced into the starting gene. In some preferred embodiments, these mutations are identified by sequencing hits identified during the screening step of the method. In most embodiments, mutations that are more frequently found in hits are likely to be improving mutations, as compared to an unscreened combinatorial consensus mutagenesis library.

As used herein, the term "enhanced combinatorial consensus mutagenesis library" refers to a CCM library that is designed and constructed based on screening and/or sequencing results from an earlier round of CCM mutagenesis and screening. In some embodiments, the enhanced CCM library is based on the sequence of an initial hit resulting from an earlier round of CCM. In additional embodiments, the enhanced CCM is designed such that mutations that were frequently observed in initial hits from earlier rounds of mutagenesis and screening are favored. In some preferred embodiments, this is accomplished by omitting primers that encode performance-reducing mutations or by increasing the concentration of primers that encode performance-enhancing mutations relative to other primers that were used in earlier CCM libraries.

As used herein, the term "performance-reducing mutations" refer to mutations in the combinatorial consensus mutagenesis library that are less frequently found in hits resulting from screening as compared to an unscreened combinatorial consensus mutagenesis library. In preferred embodiments, the screening process removes and/or reduces the abundance of variants that contain "performance-reducing mutations."

As used herein, the term "functional assay" refers to an assay that provides an indication of a protein's activity. In particularly preferred embodiments, the term refers to assay systems in which a protein is analyzed for its ability to function in its usual capacity. For example, in the case of enzymes, a functional assay involves determining the effectiveness of the enzyme in catalyzing a reaction.

As used herein, the term "target property" refers to the property of the starting gene that is to be altered. In some preferred embodiments, the target property is the stability of a gene product (e.g., resistance to denaturation, proteolysis or other degradative factors), while in other embodiments the level of production in a production host is altered.

The term "property" or grammatical equivalents thereof in the context of a nucleic acid, as used herein, refer to any characteristic or attribute of a nucleic acid that can be selected or detected. These properties include, but are not limited to, a property affecting binding to a polypeptide, a property conferred on a cell comprising a particular nucleic acid, a property affecting gene transcription (e.g., promoter strength, promoter recognition, promoter regulation, enhancer function), a property affecting RNA processing (e.g., RNA splicing, RNA stability, RNA conformation, and post-transcriptional modification), a property affecting translation (e.g., level, regulation, binding of mRNA to ribosomal proteins, post-translational modification). For example, a binding site for a transcription factor, polymerase, regulatory factor, etc., of a nucleic acid may be altered to produce desired characteristics or to identify undesirable characteristics.

The term "property" or grammatical equivalents thereof in the context of a polypeptide, as used herein, refer to any characteristic or attribute of a polypeptide that can be selected or detected. These properties include, but are not limited to oxidative stability, substrate specificity, catalytic activity, thermal stability, alkaline stability, pH activity profile, resistance to proteolytic degradation, $K_M$, $k_{cat}$, $k_{cat}/k_M$ ratio, protein folding, inducing an immune response, ability to bind to a ligand, ability to bind to a receptor, ability to be secreted, ability to be displayed on the surface of a cell, ability to oligomerize, ability to signal, ability to stimulate cell proliferation, ability to inhibit cell proliferation, ability to induce apoptosis, ability to be modified by phosphorylation or glycosylation, ability to treat disease.

As used herein, the term "screening" has its usual meaning in the art and is, in general a multi-step process. In the first step, a mutant nucleic acid or variant polypeptide therefrom is provided. In the second step, a property of the mutant nucleic acid or variant polypeptide is determined. In the third step, the determined property is compared to a property of the corresponding parent nucleic acid, to the property of the corresponding naturally occurring polypeptide or to the property of the starting material (e.g., the initial sequence) for the generation of the mutant nucleic acid.

It will be apparent to the skilled artisan that the screening procedure for obtaining a nucleic acid or protein with an altered property depends upon the property of the starting material the modification of which the generation of the mutant nucleic acid is intended to facilitate. The skilled artisan will therefore appreciate that the invention is not limited to any specific property to be screened for and that the following description of properties lists illustrative examples only. Methods for screening for any particular property are generally described in the art. For example, one can measure binding, pH, specificity, etc., before and after mutation, wherein a change indicates an alteration. Preferably, the screens are performed in a high-throughput manner, including multiple samples being screened simultaneously, including, but not limited to assays utilizing chips, phage display, and multiple substrates and/or indicators.

As used herein, in some embodiments, screens encompass selection steps in which variants of interest are enriched from a population of variants. Examples of these embodiments include the selection of variants that confer a growth advantage to the host organism, as well as phage display or any other method of display, where variants can be captured from a population of variants based on their binding or catalytic properties. In a preferred embodiment, a library of variants is exposed to stress (heat, protease, denaturation) and subsequently variants that are still intact are identified in a screen or enriched by selection. It is intended that the term encompass any suitable means for selection. Indeed, it is not intended that the present invention be limited to any particular method of screening.

As used herein, the term "targeted randomization" refers to a process that produces a plurality of sequences where one or several positions have been randomized. In some embodiments, randomization is complete (i.e., all four nucleotides, A, T, G, and C can occur at a randomized position. In alternative embodiments, randomization of a nucleotide is limited to a subset of the four nucleotides. Targeted randomization can be applied to one or several codons of a sequence, coding for one or several proteins of interest. When expressed, the resulting libraries produce protein populations in which one or more amino acid positions can contain a mixture of all 20 amino acids or a subset of amino acids, as determined by the randomization scheme of the randomized codon. In some embodiments, the individual members of a population resulting from targeted randomization differ in the number of amino acids, due to targeted or random insertion or deletion of codons. In further embodiments, synthetic amino acids are included in the protein populations produced. In some preferred embodiments, the majority of members of a population resulting from targeted randomization show greater sequence homology to the consensus sequence than the starting gene. In some embodiments, the sequence encodes one or more proteins of interest. In alternative embodiments, the proteins have differing biological functions. In some preferred embodiments, the incoming sequence comprises at least one selectable marker. This sequence can code for one or more proteins of interest. It can have other biological function(s). In many cases the incoming sequence will include a selectable marker, such as a gene that confers resistance to an antibiotic.

The terms "modified sequence" and "modified genes" are used interchangeably herein to refer to a sequence that includes a deletion, insertion or interruption of naturally occurring nucleic acid sequence. In some preferred embodiments, the expression product of the modified sequence is a truncated protein (*e.g.*, if the modification is a deletion or interruption of the sequence). In some particularly preferred embodiments, the truncated protein retains biological activity. In alternative embodiments, the expression product of the modified sequence is an elongated protein (*e.g.,* modifications comprising an insertion into the nucleic acid sequence). In some embodiments, an insertion leads to a truncated protein (*e.g.*, when the insertion results in the formation of a stop codon). Thus, an insertion may result in either a truncated protein or an elongated protein as an expression product.

As used herein, the terms "mutant sequence" and "mutant gene" are used interchangeably and refer to a sequence that has an alteration in at least one codon occurring in a host cell's wild-type sequence. The expression product of the mutant sequence is a protein with an altered amino acid sequence relative to the wild-type. The expression product may have an altered functional capacity (*e.g.*, enhanced enzymatic activity).

The terms "mutagenic primer" or "mutagenic oligonucleotide" (used interchangeably herein) are intended to refer to oligonucleotide compositions which correspond to a portion of the template sequence and which are capable of hybridizing thereto. With respect to mutagenic primers, the primer will not precisely match the template nucleic acid, the mismatch or mismatches in the primer being used to introduce the desired mutation into the nucleic acid library. As used herein, "non-mutagenic primer" or "non-mutagenic oligonucleotide" refers to oligonucleotide compositions that match precisely to the template nucleic acid. In one embodiment of the invention, only mutagenic primers are used. In another preferred embodiment of the invention, the primers are designed so that for at least one region at which a mutagenic primer has been included, there is also non-mutagenic primer included in the oligonucleotide mixture. By adding a mixture of mutagenic primers and non-mutagenic primers corresponding to at least one of the mutagenic primers, it is possible to produce a resulting nucleic acid library in which a variety of combinatorial mutational patterns are presented. For example, if it is desired that some of the members of the mutant nucleic acid library retain their parent sequence at certain positions while other members are mutant at such sites, the non-mutagenic primers provide the ability to obtain a specific level of non-mutant members within the nucleic acid library for a given residue. The methods of the invention employ mutagenic and non-mutagenic oligonucleotides which are generally between 10-50 bases in length, more preferably about 15-45 bases in length. However, it may be necessary to use primers that are either shorter than 10 bases or longer than 50 bases to obtain the mutagenesis result desired. With respect to corresponding mutagenic and non-mutagenic primers, it is not necessary that the corresponding oligonucleotides be of identical length, but only that there is overlap in the region corresponding to the mutation to be added.

In some embodiments, primers are added in a pre-defined ratio. For example, if it is desired that the resulting library have a significant level of a certain specific mutation and a lesser amount of a different mutation at the same or different site, by adjusting the amount of primer added, it is possible to produce the desired biased library. Alternatively, by adding lesser or greater amounts of non-mutagenic primers, it is possible to adjust the frequency with which the corresponding mutation(s) are produced in the mutant nucleic acid library.

As used herein, the phrase "contiguous mutations" refers to mutations that are presented within the same oligonucleotide primer. For example, contiguous mutations may be adjacent or nearby each other, however, they will be introduced into the resulting mutant template nucleic acids by the same primer.

As used herein, the phrase "discontiguous mutations" refers to mutations that are presented in separate oligonucleotide primers. For example, discontiguous mutations will be introduced into the resulting mutant template nucleic acids by separately prepared oligonucleotide primers.

The terms "wild-type sequence," "wild-type nucleic acid sequence," and "wild-type gene" are used interchangeably herein, to refer to a sequence that is native or naturally occurring in a host cell. In some embodiments, the wild-type sequence refers to a sequence of interest that is the starting point of a protein-engineering project. The wild-type sequence may encode either a homologous or heterologous protein. A homologous protein is one the host cell would produce without intervention. A heterologous protein is one that the host cell would not produce but for the intervention.

The term "oxidation stable" refers to proteases of the present disclosure that retain a specified amount of enzymatic activity over a given period of time under conditions prevailing during the proteolytic, hydrolyzing, cleaning or other process of the invention, for example while exposed to or contacted with bleaching agents or oxidizing agents.

In some embodiments, the proteases retain at least 50%, 60%, 70%, 75%, 80%, 85%, 90%, 92%, 95%, 96%, 97%, 98% or 99% proteolytic activity after contact with a bleaching or oxidizing agent over a given time period, for example, at least 1 minute, 3 minutes, 5 minutes, 8 minutes, 12 minutes, 16 minutes, 20 minutes, etc. disclosure

The term "chelator stable" refers to proteases of the present disclosure that retain a specified amount of enzymatic activity over a given period of time under conditions prevailing during the proteolytic, hydrolyzing, cleaning or other process of the disclosure, for example while exposed to or contacted with chelating agents. In some embodiments, the proteases retain at least 50%, 60%, 70%, 75%, 80%, 85%, 90%, 92%, 95%, 96%, 97%, 98% or 99% proteolytic activity after contact with a chelating agent over a given time period, for example, at least 10 minutes, 20 minutes, 40 minutes, 60 minutes, 100 minutes, etc.

The terms "thermally stable" and "thermostable" refer to proteases of the present disclosure that retain a specified amount of enzymatic activity after exposure to identified temperatures over a given period of time under conditions prevailing during the proteolytic, hydrolyzing, cleaning or other process of the disclosure, for example while exposed to altered temperatures. Altered temperatures include increased or decreased temperatures. In some embodiments, the proteases retain at least 50%, 60%, 70%, 75%, 80%, 85%, 90%, 92%, 95%, 96%, 97%, 98% or 99% proteolytic activity after exposure to altered temperatures over a given time period, for example, at least 60 minutes, 120 minutes, 180 minutes, 240 minutes, 300 minutes, etc.

The term "enhanced stability" in the context of an oxidation, chelator, thermal and/or pH stable protease refers to a higher retained proteolytic activity over time as compared to other serine proteases (*e.g.*, subtilisin proteases) and/or wild-type enzymes.

The term "diminished stability" in the context of an oxidation, chelator, thermal and/or pH stable protease refers to a lower retained proteolytic activity over time as compared to other serine proteases (e.g., subtilisin proteases) and/or wild-type enzymes.

As used herein, the term "cleaning composition" includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, car or carpet shampoos, bathroom cleaners; hair shampoos and hair-rinses; shower gels and foam baths and metal cleaners; as well as cleaning auxiliaries such as bleach additives and "stain-stick" or pre-treat types.

Unless otherwise noted, all component or composition levels are in reference to the active level of that component or composition, and are exclusive of impurities, for example, residual solvents or by-products, which may be present in commercially available sources.

Enzyme components weights are based on total active protein. All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The term "cleaning activity" refers to the cleaning performance achieved by the protease under conditions prevailing during the proteolytic, hydrolyzing, cleaning or other process of the disclosure. In some embodiments, cleaning performance is determined by the application of various cleaning assays concerning enzyme sensitive stains, for example grass, blood, milk, or egg protein as determined by various chromatographic, spectrophotometric or other quantitative methodologies after subjection of the stains to standard wash conditions. Exemplary assays include, but are not limited to those described in WO 99/34011, and U.S. Patent No. 6,605,458, as well as those methods included in the Examples.

The term "cleaning effective amount" of a protease refers to the quantity of protease described hereinbefore that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (*e.g.*, granular, bar) composition is required, etc.

The term "cleaning adjunct materials" as used herein, means any liquid, solid or gaseous material selected for the particular type of cleaning composition desired and the form of the product (*e.g.*, liquid, granule, powder, bar, paste, spray, tablet, gel; or foam composition), which materials are also preferably compatible with the protease enzyme used in the composition. In some embodiments, granular compositions are in "compact" form, while in other embodiments, the liquid compositions are in a "concentrated" form.

The term "enhanced performance" in the context of cleaning activity refers to an increased or greater cleaning activity of certain enzyme sensitive stains such as egg, milk, grass or blood, as determined by usual evaluation after a standard wash cycle and/or multiple wash cycles.

The term "diminished performance" in the context of cleaning activity refers to a decreased or lesser cleaning activity of certain enzyme sensitive stains such as egg, milk, grass or blood, as determined by usual evaluation after a standard wash cycle.

The term "comparative performance" in the context of cleaning activity refers to at least 60%, at least 70%, at least 80% at least 90% at least 95% of the cleaning activity of a comparative protease (*e.g.*, commercially available proteases). Cleaning performance can be determined by comparing the proteases of the present invention with other proteases in various cleaning assays concerning enzyme sensitive stains such as blood, milk and/or ink (BMI) as determined by usual spectrophotometric or analytical methodologies after standard wash cycle conditions.

As used herein, a "low detergent concentration" system includes detergents where less than about 800 ppm of detergent components are present in the wash water. Japanese detergents are typically considered low detergent concentration systems, as they have usually have approximately 667 ppm of detergent components present in the wash water.

As used herein, "medium detergent concentration" systems includes detergents wherein between about 800 ppm and about 2000ppm of detergent components are present in the wash water. North American detergents are generally considered to be medium detergent concentration systems as they have usually approximately 975 ppm of detergent components present in the wash water. Brazilian detergents typically have approximately 1500 ppm of detergent components present in the wash water.

As used herein, "high detergent concentration" systems includes detergents wherein greater than about 2000 ppm of detergent components are present in the wash water. European detergents are generally considered to be high detergent concentration systems as they have approximately 3000-8000 ppm of detergent components in the wash water.

As used herein, "fabric cleaning compositions" include hand and machine laundry detergent compositions including laundry additive compositions and compositions suitable for use in the soaking and/or pretreatment of stained fabrics (*e.g.,* clothes, linens, and other textile materials).

As used herein, "non-fabric cleaning compositions" include non-textile (*i.e.,* fabric) surface cleaning compositions, including but not limited to dishwashing detergent compositions, oral cleaning compositions, denture cleaning compositions, and personal cleansing compositions.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically 17-35% by weight of the total composition. In contrast, in compact compositions, the filler salt is present in amounts not exceeding 15% of the total composition. In some embodiments, the filler salt is present in amounts that do not exceed 10%, or more preferably, 5%, by weight of the composition. In some embodiments, the inorganic filler salts are selected from the alkali and alkaline-earth-metal salts of sulfates and chlorides. A preferred filler salt is sodium sulfate.

## DETAILED DESCRIPTION OF THE INVENTION

The present invention provides methods for engineering enzymes to optimize their performance under certain environmental conditions of interest. In some embodiments, the present invention provides methods for engineering enzymes to optimize their catalytic activity under particular environmental conditions. In some preferred embodiments, the present invention provides methods for engineering enzymes to optimize their catalytic activity and/or stability under adverse environmental conditions. In some preferred embodiments, the present invention provides methods for engineering enzymes to optimize their stability, particularly under adverse environmental conditions. In some preferred embodiments, the present invention provides methods for altering the net surface charge and/or surface charge distribution of enzymes (*e.g.*, metalloproteases) to obtain enzyme variants that demonstrate improved performance and/or stability in detergent formulations as compared to the starting or parent enzyme.

In some embodiments, the present invention provides methods for engineering an enzyme to simultaneously optimize its catalytic activity and stability under adverse environmental conditions, even when these two properties are negatively correlated when the effects of single mutations are analyzed. In particular, the present invention provides methods for altering the net surface charge and/or surface charge distribution of a metalloprotease to obtain enzyme variants demonstrating improved performance in detergent formulations.

Disclosed herein are methods and compositions comprising at least one variant neutral metalloprotease that has improved wash performance and/or stability in detergent formulation(s). In some embodiments, the present disclosure provides variants of the *Bacillus amyloliquefaciens* neutral metalloprotease. The present invention finds particular use in applications including, but not limited to cleaning, bleaching and disinfecting. Additionally, the present invention provides methods for engineering an enzyme to optimize its catalytic activity under adverse environmental conditions. In particular the present invention provides methods for altering the net surface charge and/or surface charge distribution of a metalloprotease to obtain enzyme variants demonstrating improved performance and/or stability in detergent formulations.

Many proteins and enzymes are highly susceptible to denaturation and undergo irreversible denaturation when stored in laundry detergents. Laundry detergents are known to contain anionic, cationic and non-ionic surfactants where the surfactant is classified by their ionic (electrical charge) properties in water. These ingredients interact with the surface charge of a protein molecule resulting in protein denaturation (*e.g.*, loss of structure and function). NprE (a neutral metalloprotease) has been shown to be unstable when stored in a detergent formulation including a surfactant such as LAS. LAS is an anionic surfactant where the overall negative charge enhances an interaction with the positively charged side chains of amino acids located on a protein surface. Such electrostatic interactions affect the intrinsic stability of a protein by weakening or disrupting stabilizing electrostatic interactions. The destabilized protein then unfolds and becomes inactive. During the development of the present invention, the surface charge of the enzyme was found to profoundly influence wash performance and/or detergent stability. Additionally, the distribution of charged residues on a protease surface was found to strongly affect wash performance and/or stability. The protein engineering methods of the present invention efficiently optimize proteases for enhanced performance in one or more properties in detergent formulations, by optimizing the net surface charge and/or surface charge distribution.

Briefly, in some embodiments of the present invention the methods involve creation of site-evaluation libraries at a number of amino-acid residues in an enzyme of interest and assaying the variant enzymes for the properties of interest. This allows the identification of beneficial, neutral, and detrimental mutations as well as the optimal charge change (relative to the parent enzyme) for the propert(ies) of interest. In some alternative embodiments, charge scans of all the residues to generate variants with mutations that alter charge at each site (*e.g.*, mutate neutral residues to positive and/or negative charges, and mutate charged residues to oppositely charged and/or neutral residues. In some further preferred embodiments, the methods involve creating combinatorial "charge-balanced" libraries of variants, which include beneficial mutations that change the enzyme charge in the desired direction and beneficial or neutral mutations that change the charge in the opposite direction, and then assaying the charge-balanced library for the propert(ies) of interest. Thus, the surface charge of the enzyme and the surface charge distribution are simultaneously optimized, and it is possible to identify enzyme variants having improvements in multiple properties.

The methods of the present invention find use in improving the performance of various classes of enzymes as well as proteases (*e.g.*, amylases, cellulases, oxidases, cutinases, mannanases, pectinases, amylases, lipases. etc). In addition, the disclosed method may find use in the optimization of non-enzymatic protein properties which require a particular surface charge and charge distribution (*e.g.*, expression, cell-surface binding, amenability to formulation, etc.).

## I. Production of Protease Variants With Improved Properties

A large number of site-evaluation libraries were constructed for NprE in which every amino acid of the mature protein was replaced with most of the other amino acids *(See,* U.S. Patent No. 8,535,927 and WO 2005/052146). These libraries were screened for detergent stability and BMI cleaning performance. The screening data were then analyzed with respect to the effect of charge alteration conferred by the mutations. Both increased stability and good BMI (blood, milk, ink) cleaning performance are desirable in engineered NprE variants, yet they initially appeared to be mutually exclusive properties. The present invention provides means to produce a more stable variant that exhibits good BMI cleaning performance.

Thus, the present invention provides methods to identify mutations that give elevated stability or BMI cleaning performance without unduly sacrificing the other parameter. As used herein the phrase "unduly unfavorable" refers to protein properties having less than desired values. This term encompasses some low performing proteins having neutral mutations (less than 80% of the performance value of the parent or wild type protein); poor performing proteins having non-deleterious mutations (less than 50%); and essentially inactive proteins having deleterious mutations (less than 5%). In some embodiments, the relative performance values are expressed as a performance index (PI), which is the ratio of variant protein performance to parent protein performance. Then, the charge mutations are balanced, so that the final variant is +1 to +3 relative to the wild-type enzyme. In addition, the present invention provides means to select amino acid residues which appear to be non-interacting in the 3-D structure of the enzyme, thereby minimizing non-additivity between multiple mutations.

As described herein, four NprE variants were constructed using the methods of the present invention. These variants contained from ten to eighteen mutations. As described in greater detail in the Examples, these variants demonstrated increased stability and BMI cleaning performance similar to the wild-type enzyme.

## II. General Methods for Production of Beneficial Enzyme Variants

As described herein, a relationship between wash performance in a BMI microswatch assay and the overall charge on the surface of an enzyme was determined. The methods of the present invention find use in improving the performance of various enzymes and proteins (*e.g.*, amylases, cellulases, oxidases, cutinases, mannanases, pectinases lipases, proteases, and other enzymes). Briefly, amino acid residues located on the surface of a wild-type enzyme that are greater than about 25% exposed to solvent, greater than about 50% exposed to solvent, or greater than about 65 % exposed to solvent are identified, and site-evaluation libraries, where each wild-type residue is substituted with a plurality of other naturally occurring amino acids, are created. In some embodiments, protein engineering at the surface of a molecule involves the replacement of neutral amino acid side chains with acidic or basic side chains and/or replacing positively charged side chains with neutral or negatively charged side chains or vice versa. In addition, the net charge change of the variant enzymes that show improved wash performance on BMI are noted, in order to define this structure-function relationship. In additional embodiments, once the optimum charge is determined for a given enzyme, natural isolates are screened, in order to identify enzyme variants with the optimum charge/charge distribution.

### III. Production of Amylase Variants with Improved Properties

A combinatorial charge library was constructed for AmyS-S242Q by introducing combinations of substitutions in four positions in the mature enzyme. This library was screened for BODIPY starch hydrolysis, rice starch microswatch cleaning performance, and enzyme expression. The screening data were then analyzed with respect to the effect of charge alteration conferred by the mutations. Both increased protein expression and good enzyme performance are desirable in engineered AmyS-S242Q variants, yet these properties were found to be negatively correlated. The present invention provides means to produce a more highly expressed variant that exhibits good rice starch cleaning performance. Thus, the present invention provides methods to identify mutations that give increased expression or enzymatic activity without unduly sacrificing the other parameter.

### EXPERIMENTAL

The following examples are provided in order to demonstrate and further illustrate certain preferred embodiments and aspects of the present invention and are not to be construed as limiting the scope thereof.

In the experimental disclosure which follows, the following abbreviations apply: °C (degrees Centigrade); rpm (revolutions per minute); $H_2O$ (water); HCl (hydrochloric acid); aa and AA (amino acid); bp (base pair); kb (kilobase pair); kD (kilodaltons); gm (grams); $\mu$g and ug (micrograms); mg (milligrams); ng (nanograms); $\mu$l and ul (microliters); ml (milliliters); mm (millimeters); nm (nanometers); $\mu$m and um (micrometer); M (molar); mM (millimolar);$\mu$ M and uM (micromolar); U (units); V (volts); MW (molecular weight); sec (seconds); min(s) (minute/minutes); hr(s) (hour/hours); $MgCl_2$ (magnesium chloride); NaCl (sodium chloride); $OD_{280}$ (optical density at 280 nm); $OD_{405}$ (optical density at 405 nm); $OD_{600}$ (optical density at 600 nm); PAGE (polyacrylamide gel electrophoresis); EtOH (ethanol); PBS (phosphate buffered saline [150 mM NaCl, 10 mM sodium phosphate buffer, pH 7.2]); LAS (lauryl sodium sulfonate); SDS (sodium dodecyl sulfate); Tris (tris(hydroxymethyl)aminomethane); TAED (N,N,N'N'-tetraacetylethylenediamine); BES (polyesstersulfone); MES (2-morpholinoethanesulfonic acid, monohydrate; f.w. 195.24; Sigma # M-3671); $CaCl_2$ (calcium chloride, anhydrous; f.w. 110.99; Sigma # C-4901); DMF (N,N-dimethylformamide, f.w. 73.09, d = 0.95); Abz-AGLA-Nba (2-Aminobenzoyl-L-alanylglycyl-L-leucyl-L-alanino-4-nitrobenzylamide, f.w. 583.65; Bachem # H-6675, VWR catalog # 100040-598); SBG1% ("Super Broth with Glucose"; 6 g Soytone [Difco], 3 g yeast extract, 6 g NaCl, 6 g glucose); w/v (weight to volume); v/v (volume to volume); Npr and npr (neutral metalloprotease); SEQUEST® (SEQUEST database search program, University of Washington); *Npr* and *npr* (neutral metalloprotease gene); nprE and NprE (*B. amyloliquefaciens* neutral metalloprotease); PMN (purified MULTIFECT® metalloprotease); MTP (microtiter plate); MS (mass spectroscopy); SRI (Stain Removal Index); TIGR (The Institute for Genomic Research, Rockville, MD); AATCC (American Association of Textile and Coloring Chemists); Procter & Gamble (Procter & Gamble, Inc., Cincinnati, OH); Beckman (Beckman Coulter, Inc., Fullerton, CA); Amersham (Amersham Life Science, Inc. Arlington Heights, IL); ICN (ICN Pharmaceuticals, Inc., Costa Mesa, CA); Pierce (Pierce Biotechnology, Rockford, IL); EMPA (Eidgenossische Material Prufungs und Versuch Anstalt, St. Gallen, Switzerland); CFT (Center for Test Materials, Vlaardingen, The Netherlands); Amicon (Amicon, Inc., Beverly, MA); ATCC (American Type Culture Collection, Manassas, VA); Becton Dickinson (Becton Dickinson Labware, Lincoln Park, NJ); Perkin-Elmer (Perkin-Elmer, Wellesley, MA); Rainin (Rainin Instrument, LLC, Woburn, MA); Eppendorf (Eppendorf AG, Hamburg, Germany); Waters (Waters, Inc., Milford, MA); Geneart (Geneart GmbH, Regensburg, Germany); Perseptive Biosystems (Perseptive Biosystems, Ramsey, MN); Molecular Probes (Molecular Probes, Eugene, OR); BioRad (BioRad, Richmond, CA); Clontech (CLONTECH Laboratories, Palo Alto, CA); Difco (Difco Laboratories, Detroit, MI); GIBCO BRL or Gibco BRL (Life Technologies, Inc., Gaithersburg, MD); Epicentre (Epicentre Biotechnologies, Madison, WI); Zymo Research (Zymo Research Corp., Orange, CA); Integrated DNA Technologies (Integrated DNA Technologies, Inc., Coralville, IA): New Brunswick (New Brunswick Scientific Company, Inc., Edison, NJ); Thermoelectron (Thermoelectron Corp., Waltham, MA); BMG (BMG Labtech, GmbH, Offenburg, Germany); Greiner (Greiner Bio-One, Kremsmuenster, Austria); Novex (Novex, San Diego, CA); Finnzymes (Finnzymes OY, Finland) Qiagen (Qiagen, Inc., Valencia, CA); Invitrogen (Invitrogen Corp., Carlsbad, CA); Sigma (Sigma Chemical Co.,

St. Louis, MO); DuPont Instruments (Asheville, NY); Global Medical Instrumentation or GMI (Global Medical Instrumentation; Ramsey, MN); MJ Research (MJ Research, Waltham, MA); Infors (Infors AG, Bottmingen, Switzerland); Stratagene (Stratagene Cloning Systems, La Jolla, CA); Roche (Hoffmann La Roche, Inc., Nutley, NJ); Ion Beam Analysis Laboratory (Ion Bean Analysis Laboratory, The University of Surrey Ion Beam Centre (Guildford, UK); TOM (Terg-o-Meter); BMI (blood, milk, ink); BaChem (BaChem AG, Bubendorf, Switzerland); Molecular Devices (Molecular Devices, Inc., Sunnyvale, CA); MicroCal (Microcal, Inc., Northhampton, MA); Chemical Computing (Chemical Computing Corp., Montreal, Canada); NCBI (National Center for Biotechnology Information); GE Healthcare (GE Healthcare, UK).

## EXAMPLE 1

### Assays

The following assays were used in the examples described below. Any deviations from the protocols provided below are indicated in the examples. In these experiments, a spectrophotometer was used to measure the absorbance of the products formed after the completion of the reactions. A reflectometer was used to measure the reflectance of the swatches.

### A. Protein Content Determination

#### 1. BCA (bicinchoninic acid) Assay for Protein Content Determination

In these assays, BCA (Pierce) assay was used to determine the protein concentration in protease samples on a microtiter plate (MTP) scale. In this assay system, the chemical and reagent solutions used were: BCA protein assay reagent, and Pierce Dilution buffer (50 mM MES, pH 6.5, 2mM $CaCl_2$, 0.005% TWEEN®-80). The equipment used was a SpectraMAX (type 340) MTP reader. The MTPs were obtained from Costar (type 9017).

In the test, 200 μl BCA reagent was pipetted into each well, followed by 20 μl diluted protein. After thorough mixing, the MTPs were incubated for 30 minutes at 37°C. Air bubbles were removed, and the optical density (OD) of the solution within the wells was read at 562 nm. To determine the protein concentration, the background reading was subtracted from the sample readings. The $OD_{562}$ values were plotted for protein standards (purified protease), to produce a standard curve. The protein concentrations of the samples were extrapolated from the standard curve.

#### 2. Bradford Assay for Protein Content Determination

In these assays, the Bradford dye reagent (Quick Start) assay was used to determine the protein concentration in protease samples on a MTP scale.

In this assay system, the chemical and reagent solutions used were: Quick Start Bradford Dye Reagent (BIO-RAD Catalog No. 500-0205), dilution buffer (10mM NaCl, 0.1 mM CaCl2, 0.005% TWEEN®-80). The equipment used was a Biomek FX Robot (Beckman) and a SpectraMAX (type 340; Molecular Devices) MTP reader. The MTPs were from Costar (type 9017).

In the test, 200 μl Bradford Dye Reagent was pipetted into each well, followed by 15 μl dilution buffer. Finally, 10 μl of filtered culture broth were added to the wells.

After thorough mixing, the MTPs were incubated for at least 10 minutes at room temperature. Air bubbles were blown away and the ODs of the wells were read at 595 nm. To determine the protein concentration, the background reading (*i.e.,* from uninoculated wells) was subtracted from the sample readings. The obtained $OD_{595}$ values provide a relative measure of the protein content in the samples.

### B. Microswatch Assay for Testing Protease Performance

The equipment used included an Eppendorf Thermomixer and a SpectraMAX (type 340) MTP reader. The MTPs were obtained from Costar (type 9017).

Detergent Preparation (TIDE® 2X Ultra, Clean Breeze liquid laundry detergent (Procter & Gamble); US wash conditions)

Milli-Q water was adjusted to 6 gpg water hardness (Ca/Mg=3/l), and 0.78 g/l TIDE® 2X Ultra Clean Breeze" detergent was added. The detergent had been previously heat-treated at 95 °C for one hour to inactivate any enzymes present in the formulation. The detergent solution was stirred for 15 minutes. Then, 5 mM HEPES (free acid) was added and the pH adjusted to 8.2.

Microswatches

Microswatches of 0.25 inch circular diameter were obtained from CFT Vlaardingen. Before cutting of the swatches, the fabric (EMPA 116) was washed with water. One microswatch was placed in each well of a 96-well microtiter plate.

Test Method

The desired detergent solution was prepared as described above. After equilibrating the Thermomixer at 25°C, 190 $\mu$l of detergent solution was added to each microswatch-containing well of the MTP. To this mixture, 10 $\mu$l of the diluted enzyme solution was added so that the final enzyme concentration was 1 $\mu$g/ml (determined from BCA assay). The MTP was sealed with tape and placed in the incubator for 30 minutes, with agitation at 1400 rpm. Following incubation under the appropriate conditions, 100 $\mu$l of the solution from each well was transferred into a fresh MTP. The new MTP containing 100 $\mu$l of solution/well was read at 405 nm using a MTP SpectraMax reader. Blank controls, as well as a control containing a microswatch and detergent but no enzyme were also included.

Calculation of the BMI Performance

The obtained absorbance value was corrected for the blank value (*i.e.*, obtained after incubation of microswatches in the absence of enzyme). The resulting absorbance provided a measure of the hydrolytic activity of the tested enzyme.

**C**. **TIDE® Stability Assay**

The stability of wild type and variants proteases was measured after an incubation step in the presence of 25% heat-treated TIDE® 2x Ultra Clean Breeze" liquid laundry detergent. The initial and residual activity was determined using the AGLA-assay described below. Equipment used included a fluorescence 96-well plate reader, an incubator/shaker (iEMS; Thermoelectron) and an incubator/shaker (Innova; New Brunswick (type 4230). The MTPs were from Costar (type 9017) and from Greiner (black plates, type 655076).

Chemicals and Reagents:

In this assay system, the chemical and reagent solutions used were:

TIDE® 2x Ultra Clean Breeze
125 g TIDE® 2x Ultra Clean Breeze (heat-treated at 95°C as above) dissolved in a mixture of 50 g of 50 mM HEPES pH 8.2 and 275 ml water; the TIDE® concentration was 27.7%, after dilution with supernatant 25 % (referred to below as "TIDE®")
MES dilution buffer
52.6 mM MES/NaOH, 2.6 mM $CaCl_2$, 0.005% TWEEN®-80, pH 6.5
AGLA substrate
BaChem, Catalog No. H-6675 or American Peptide Co., Catalog No. 81-0-31
AGLA substrate solution
451 mg of AGLA dissolved in 16 ml N,N dimethylformamide; this solution was poured into 304 ml of MES-buffer (52.6 mM MES/NaOH, 2.6 mM $CaCl_2$, 0.005% TWEEN®-80, pH 6.5) with stirring

Test Methods:

Unstressed Conditions:

First, 20 $\mu$l filtered culture broth was diluted with 180 $\mu$l MES dilution buffer. Then, 20 $\mu$l of this diluted broth was diluted with 180 $\mu$l MES dilution buffer. Then, 10 $\mu$l of this dilution was diluted with 190 $\mu$l AGLA-substrate solution in a pre-warmed plate at 25°C. Any air bubbles present were blown away and the plate was measured according to the AGLA protease assay protocol.

Stressed Conditions:

First, 20 $\mu$l filtered culture broth was diluted with 180 $\mu$l TIDE® detergent solution and after premixing in the iEMS shaker for 5 minutes, were incubated further in the Innova shaker. The plate was incubated for a total of 60 minutes at 32°C, at 200 rpm. In addition, 20 μl filtered culture broth were diluted with 180 μl TIDE® detergent solution and after premixing in the iEMS shaker for 5 minutes, were incubated further in the Innova shaker. The plate was incubated for a total of 40 minutes at 20°C, at 200 rpm. Then, 20 μl of either of these solutions were diluted with 180 μl MES dilution buffer and 10 μl of this dilution were diluted with 190 μl AGLA-substrate solution in a pre-warmed plate at 25°C. Any air bubbles present were blown away and the plate was measured according to the AGLA protease assay protocol.

Calculations

Fluorescence measurements were taken at excitation of 350 nm and emission of 415 nm. The spectrofluorometer software calculated the reaction rates of the increase in fluorescence for each well to a linearly regressed line of milli-RFU / min:

$$\text{Percentage of residual activity:} \quad \frac{(\text{Slope of stressed condition}) * 100}{(\text{Slope of unstressed condition})}$$

### D. 2-Aminobenzoyl-L-alanylglycyl-L-leucyl-L-alanino-4-nitrobenzylamide Protease Assay (Abz-AGLA-Nba)

The method provided below provides a degree of technical detail that yields reproducible protease assay data independent of time and place. While the assay is adaptable to given laboratory conditions, any data obtained through a modified procedure must be reconciled with results produced by the original method.

Neutral metalloproteases cleave the peptide bond between glycine and leucine of 2-aminobenzoyl-L-alanylglycyl-L-leucyl-L-alanino-4-nitrobenzylamide (Abz-AGLA-Nba). Free 2-aminobenzoyl-L-alanylglycine (Abz-AG) in solution has a fluorescence emission maximum at 415 nm with an excitation maximum of 340 nm. Fluorescence of Abz-AG is quenched by nitrobenzylamide in the intact Abz-AGLA-Nba molecule.

In these experiments, the liberation of Abz-AG by protease cleavage of Abz-AGLA-Nba was monitored by fluorescence spectroscopy (Ex. 340 / Em. 415). The rate of appearance of Abz-AG was a measure of proteolytic activity. Assays were performed under non-substrate limited initial rate conditions.

A microplate mixer with temperature control (e.g., EppendorfThermomixer) was required for reproducible assay results. The assay solutions were incubated to desired temperature (e.g., 25°C) in the microplate mixer prior to enzyme addition. Enzyme solutions were added to the plate in the mixer, mixed vigorously and rapidly transferred to the plate reader.

A spectrofluorometer with capability of continuous data recording, and linear regression analysis along with temperature control was required (e.g., SpectraMax M5, Gemini EM, Molecular Devices). The reader was always maintained at the desired temperature (e.g., 25°C). The reader was set for top-read fluorescence detection and the excitation was set to 350 nm and emission to 415 nm without the use of a cut-off filter. The PMT was set to medium sensitivity and 5 readings per well. Autocalibration was turned on, but only to calibrate before the first reading. The assay was measured for 3 minutes with the reading interval minimized according to the number of wells selected to be monitored. The reader was set to calculate the rate of milli-RFU/min (thousandths of relative fluorescence units per minute). The number of readings used to calculate the rate (Vmax points) was set to the number equivalent to 2 minutes, as determined by the reading interval (*e.g.*, a reading every 10 seconds would use 12 points to calculate the rate). The max RFU was set to 50,000.

All pipetting of enzyme and substrate stock solutions was done with positive displacement pipets (Rainin Microman). Buffer, assay, and enzyme working solutions were pipetted by single or multi-channel air-displacement pipets (Rainin LTS) from tubes, reagent reservoirs or stock microplates. A repeater pipet (Eppendorf) is useful in transferring the assay solution to microplate wells when only a few wells are used, to minimize reagent loss. Automated pipetting instruments such as the Beckman FX or Cybio Cybi-well also are useful in transferring enzyme solutions from a working stock microplate to the assay microplate in order to simultaneously inoculate an entire microplate.

Reagents and Solutions:

52.6 mM MES/NaOH, 2.6 mM $CaCl_2$, pH 6.5 - MES Buffer

MES acid (10.28 g) and 292 mg anhydrous $CaCl_2$ were dissolved in approximately 900mL purified water. The solution was titrated with NaOH to pH 6.5 (at 25°C or with temperature adjustment pH probe). The pH-adjusted buffer was made up to 1L total volume. The final solution was filtered through a 0.22 μm sterile filter and kept at room temperature.

EP 2 171 057 B1

48 mM Abz-AGLA-Nba in DMF - Abz-AGLA-Nba Stock

Approximately 28 mg of Abz-AGLA-Nba was placed in a small tube. It was dissolved in DMF (volume varies, depending upon Abz-AGLA-Nba massed) and vortexed for several minutes. The solution was stored at room temperature shielded from light.

50 mM MES, 2.5 mM CaCl$_2$, 5% DMF, 2.4 mM Abz-AGLA-Nba pH 6.5 - Assay Solution

One mL Abz-AGLA-Nba stock was added to 19 mL MES Buffer and vortexed. The solution was stored at room temperature shielded from light.

50 mM MES, 2.5 mM CaCl$_2$, pH 6.5 - Enzyme Dilution Buffer

This buffer was produced by adding 5 mL purified water to 95 mL MES Buffer.

50 mM MES, 2.5 mM CaCl$_2$, 5% DMF, pH 6.5 - Substrate Dilution Buffer

Five mL pure DMF were added to 95 mL MES Buffer. This buffer was used to determine kinetic parameters.

Enzyme Solutions

The enzyme stock solutions were diluted with enzyme dilution buffer to a concentration of approximately 1 ppm. MULTIFECT® neutral protease (wild-type NprE) was diluted to concentrations below 6 ppm. Serial dilutions were preferred. Solutions were stable at room temperature for 1 hour, but for longer storage periods, the solutions were maintained on ice.

Procedure

First, all buffers, stock, and working solutions were prepared. Each enzyme dilution was assayed in triplicate, unless otherwise indicated. When not completely full, the enzyme working solution stock microplate was arranged in full vertical columns starting from the left of the plate (to accommodate the plate reader). The corresponding assay plate was similarly set up. The microplate spectrofluorometer was set up as previously described.
First, a 200 μL aliquot of assay solution was placed in the wells of a 96-well microplate. The plate was incubated for 10 min at 25°C in a temperature controlled microplate mixer, shielded from light. The assay was initiated by transferring 10 uL of the working enzyme solutions from the stock microplate to the assay microplate in the mixer. Optimally, 96-well pipetting head was used, or in some experiments, an 8-well multi-channel pipet was used to transfer from the left-most column first. The solutions were vigorously mixed for 15 seconds (900rpm in Eppendorf Thermomixer). Immediately, the assay microplate was transferred to the microplate spectrofluorometer and recording of fluorescence measurements at excitation of 350 nm and emission of 415 nm were begun. The spectrofluorometer software calculated the reaction rates of the increase in fluorescence for each well to a linearly regressed line of milli-RFU / min. In some experiments, a second plate was placed in the microplate mixer for temperature equilibration while the first plate was being read.
The rate initial velocities were linear with respect to product concentration (*i.e.*, liberated 2-aminobenzoyl fluorescence) up to 0.3 mM product, which corresponded to approximately 50,000 RFU in a solution starting at 2.3mM Abz-AGLA-Nba with background fluorescence of approximately 22,000 RFU. Abz-AGLA-Nba was dissolved in DMF and was used the day it was prepared.

**EXAMPLE 2**

**NprE Protease Production in *B. subtilis***

In this Example, experiments conducted to produce NprE protease in *B. subtilis* are described. In particular, the methods used in the transformation of plasmid pUBnprE into *B. subtilis* are provided. Transformation was performed as known in the art (*See e.g.,* WO 02/14490, and US Pat. Appln. Ser. No. 11/581,102). The DNA sequence (nprE leader, nprE pro and nprE mature DNA sequence from *B.amyloliguefaciens*) provided below encodes the NprE precursor protein.

GTGGGTTTAGGTAAGAAATTGTCTGTTGCTGTCGCCGCTTCCTTTATGAGTTTAACC
ATCAGTCTGCCGGGTGTTCAGGCCGCTGAGAATCCTCAGCTTAAAGAAAACCTGAC
GAATTTTGTACCGAAGCATTCTTTGGTGCAATCAGAATTGCCTTCTGTCAGTGACAA
AGCTATCAAGCAATACTTGAAACAAACGGCAAAGTCTTTAAAGGCAATCCTTCTG
AAAGATTGAAGCTGATTGACCAAACGACCGATGATCTCGGCTACAAGCACTTCCGT
TATGTGCCTGTCGTAAACGGTGTGCCTGTGAAAGACTCTCAAGTCATTATTCACGTC

GATAAATCCAACAACGTCTATGCGATTAACGGTGAATTAAACAACGATGTTTCCGC
CAAAACGGCAAACAGCAAAAAATTATCTGCAAATCAGGCGCTGGATCATGCTTATA
AAGCGATCGGCAAATCACCTGAAGCCGTTTCTAACGGAACCGTTGCAAACAAAAAC
AAAGCCGAGCTGAAAGCAGCAGCCACAAAAGACGGCAAATACCGCCTCGCCTATG
ATGTAACCATCCGCTACATCGAACCGGAACCTGCAAACTGGGAAGTAACCGTTGAT
GCGGAAACAGGAAAAATCCTGAAAAAGCAAACAAAGTGGAGCATGCCGCCACAA
CCGGAACAGGTACGACTCTTAAAGGAAAAACGGTCTCATTAAATATTTCTTCTGAA
AGCGGCAAATATGTGCTGCGCGATCTTTCTAAACCTACCGGAACACAAATTATTAC
GTACGATCTGCAAAACCGCGAGTATAACCTGCCGGGCACACTCGTATCCAGCACCA
CAAACCAGTTTACAACTTCTTCTCAGCGCGCTGCCGTTGATGCGCATTACAACCTCG
GCAAAGTGTATGATTATTTCTATCAGAAGTTTAATCGCAACAGCTACGACAATAAA
GGCGGCAAGATCGTATCCTCCGTTCATTACGGCAGCAGATACAATAACGCAGCCTG
GATCGGCGACCAAATGATTTACGGTGACGGCGACGGTTCATTCTTCTCACCTCTTTC
CGGTTCAATGGACGTAACCGCTCATGAAATGACACATGGCGTTACACAGGAAACAG
CCAACCTGAACTACGAAAATCAGCCGGGCGCTTTAAACGAATCCTTCTCTGATGTAT
TCGGGTACTTCAACGATACTGAGGACTGGGATATCGGTGAAGATATTACGGTCAGC
CAGCCGGCTCTCCGCAGCTTATCCAATCCGACAAAATACGGACAGCCTGATAATTTC
AAAAATTACAAAAACCTTCCGAACACTGATGCCGGCGACTACGGCGGCGTGCATAC
AAACAGCGGAATCCCGAACAAAGCCGCTTACAATACGATTACAAAAATCGGCGTGA
ACAAAGCGGAGCAGATTTACTATCGTGCTCTGACGGTATACCTCACTCCGTCATCAA
CTTTTAAAGATGCAAAAGCCGCTTTGATTCAATCTGCGCGGGACCTTTACGGCTCTC
AAGATGCTGCAAGCGTAGAAGCTGCCTGGAATGCAGTCGGATTGTAA (SEQ ID
NO:1)

In the above sequence, bold indicates the DNA that encodes the mature NprE protease, standard font indicates the leader sequence (nprE leader), and underlined indicates the pro sequences (nprE pro). The amino acid sequence (NprE leader, NprE pro and NprE mature DNA sequence) provided below (SEQ ID NO:2), corresponds to the full length NprE protein. In this sequence, underlined indicates the pro sequence and bold indicates the mature NprE protease.

MGLGKKLSVAVAASFMSLTISLPGVQAAENPQLKENLTNFVPKHSLVQSELPSVSDKAI
KQYLKQNGKVFKGNPSERLKLIDQTTDDLGYKHFRYVPVVNGVPVKDSQVIIHVDKSN
NVYAINGELNNDVSAKTANSKKLSANQALDHAYKAIGKSPEAVSNGTVANKNKAELK

AAATKDGKYRLAYDVTIRYIEPEPANWEVTVDAETGKILKKQNKVEHAATTGTGTTLK
GKTVSLNISSESGKYVLRDLSKPTGTQIITYDLQNREYNLPGTLVSSTTNQFTTSSQRAA
VDAHYNLGKVYDYFYQKFNRNSYDNKGGKIVSSVHYGSRYNNAAWIGDQMIYGDGD
GSFFSPLSGSMDVTAHEMTHGVTQETANLNYENQPGALNESFSDVFGYFNDTEDWDIG
EDITVSQPALRSLSNPTKYGQPDNFKNYKNLPNTDAGDYGGVHTNSGIPNKAAYNTITK
IGVNKAEQIYYRALTVYLTPSSTFKDAKAALIQSARDLYGSQDAASVEAAWNAVGL
(SEQ ID NO:2)

The mature NprE sequence is set forth as SEQ ID NO:3. This sequence was used as the basis for making the variant libraries described herein.

AATTGTGTTLKGKTVSLNISSESGKYVLRDLSKPTGTQIITYDLQNREYNLPGTL
VSSTTNQFTTSSQRAAVDAHYNLGKVYDYFYQKFNRNSYDNKGGKIVSSVHYGSRYN
NAAWIGDQMIYGDGDGSFFSPLSGSMDVTAHEMTHGVTQETANLNYENQPGALNESFS
DVFGYFNDTEDWDIGEDITVSQPALRSLSNPTKYGQPDNFKNYKNLPNTDAGDYGGVH
TNSGIPNKAAYNTITKIGVNKAEQIYYRALTVYLTPSSTFKDAKAALIQSARDLYGSQDA
ASVEAAWNAVGL (SEQ ID NO:3)

The pUBnprE expression vector was constructed by amplifying the *nprE* gene from the chromosomal DNA of *B. amyloliquefaciens* by PCR using two specific primers:

Oligo AB1740: CTGCAGGAATTCAGATCTTAACATTTTTCCCCTATCATTTTTCCCG (SEQ ID NO:4); and
Oligo AB1741: GGATCCAAGCTTCCCGGGAAAAGACATATATGATCATGGTGAAGCC (SEQ ID NO:5)

PCR was performed in a thermocycler with Phusion High Fidelity DNA polymerase (Finnzymes). The PCR mixture contained 10 µl 5x buffer (Finnzymes Phusion), 1 µl 10mM dNTP's, 1.5µl DMSO, 1µl of each primer, 1µl Finnzymes Phusion DNA polymerase, 1µl chromosomal DNA solution 50ng/µl, 34.5 µl MilliQ water. The following PCR protocol was used: 1) 30 sec at 98°C; 2) 10 sec at 98°C; 3) 20 sec at 55°C; 4) 1 min at 72°C; 5) 25 cycles of steps 2 to 4; and 6) 5 min at 72°C.

This resulted in a 1.9 kb DNA fragment, which was digested using *Bgl*II and *Bcl*I DNA restriction enzymes. The multicopy *Bacillus* vector pUB110 (*See e.g.,* Gryczan, J Bacteriol, 134:318-329 [1978]) was digested with *Bam*HI. The PCR fragment x *Bgl*II x *Bcl*I was then ligated in the pUB110 x *Bam*HI vector to form pUBnprE expression vector.

pUBnprE was transformed to a *B. subtilis* (Δ*aprE, ΔnprE, oppA, ΔspoIIE, degUHy32, ΔamyE::(xylR,pxylA-comK)* strain. Transformation into *B. subtilis* was performed as described in WO 02/14490). Selective growth of *B. subtilis* transformants harboring the pUBnprE vector was obtained in shake flasks containing 25 ml MBD medium (a MOPS based defined medium), with 20 mg/L neomycin. MBD medium was made essentially as known in the art *(See,* Neidhardt et al., J Bacteriol, 119: 736-747 [1974]), except that $NH_4Cl_2$, $FeSO_4$, and $CaCl_2$ were left out of the base medium, 3 mM $K_2HPO_4$ was used, and the base medium was supplemented with 60 mM urea, 75 g/L glucose, and 1 % soytone. Also, the micronutrients were made up as a 100 X stock containing in one liter, 400 mg $FeSO_4 .7H_2O$, 100 mg $MnSO_4 .H_2O$, 100 mg $ZnSO_4.7H_2O$ 50 mg $CuCl_2.2H_2O$, 100 mg $CoCl_2.6H_2O$, 100 mg $NaMoO_4.2H_2O$, 100 mg $Na_2B_4O_7.10H_2O$, 10 ml of 1M $CaCl_2$, and 10 ml of 0.5 M sodium citrate. The culture was incubated for three days at 37°C in an incubator/shaker (Infors). This culture resulted in the production of secreted NprE protease with proteolytic activity as demonstrated by protease assays. Gel analysis was performed using NuPage Novex 10% Bis-Tris gels (Invitrogen, Catalog No. NP0301BOX). To prepare samples for analysis, 2 volumes of supernatant were mixed with 1 volume 1M HCl, 1 volume 4xLDS sample buffer (Invitrogen, Catalog No. NP0007), and 1% PMSF (20 mg/ml) and subsequently heated for 10 minutes at 70°C. Then, 25 μL of each sample were loaded onto the gel, together with 10 μL of SeeBlue plus 2 pre-stained protein standards (Invitrogen, Catalog No.LC5925). The results clearly demonstrated that the nprE cloning strategy described in this Example is suitable for production of active NprE in B. *subtilis.*

**EXAMPLE 3**

**Generation of Site Evaluation Libraries (SELs)**

In this Example, methods used in the construction of *nprE* SELs are described.

The pUBnprE vector, containing the nprE expression cassette described above, served as template DNA. This vector contains a unique *Bgl*II restriction site, which was utilized in the site evaluation library construction. Briefly, to construct a nprE site evaluation library, three PCR reactions were performed, including two mutagenesis PCRs to introduce the mutated codon of interest in the mature nprE DNA sequence and a third PCR used to fuse the two mutagenesis PCRs in order to construct the pUBnprE expression vector including the desired mutated codon in the mature nprE sequence.

The method of mutagenesis was based on the codon-specific mutation approach, in which the creation of all possible mutations at a time in a specific DNA triplet was performed using a forward and reverse oligonucleotide primer with a length of 25 to 45 nucleotides enclosing a specific designed triple DNA sequence NNS (N = A, C, T or G; and S = C or G) that corresponded with the sequence of the codon to be mutated and guaranteed random incorporation of nucleotides at that specific nprE mature codon. The number listed in the primer names corresponds with the specific nprE mature codon position. Sites evaluated included: 4, 12, 13, 14, 23, 24, 33, 45, 46, 47, 49, 50, 54, 58, 59, 60, 65, 66, 87, 90, 96, 97, 100, 186, 196, 211, 214, 228 and 280. An exemplary listing of primer sequences is described in US Pat. Appln. Ser. No. 11/581,102).

Two additional primers used to construct the site evaluation libraries contained the *Bgl*II restriction site together with a part of the pUBnprE DNA sequence flanking the *Bgl*II restriction site. These primers were produced by Invitrogen (50 nmole scale, desalted): pUB-BglII-FW GTCAGTCAGATCTTCCTTCAGGTTATGACC (SEQ ID NO:6); and pUB-BglII-RV GTCTCGAAGATCTGATTGCTTAACTGCTTC (SEQ ID NO:7).

Construction of each SEL started with two primary PCR amplifications using the pUB-BglII-FW primer and a specific nprE reverse mutagenesis primer. For the second PCR, the pUB-BglII -RV primer and a specific nprE forward mutagenesis primer (equal nprE mature codon positions for the forward and reverse mutagenesis primers) were used.

The introduction of the mutations in the mature nprE sequence was performed using Phusion High-Fidelity DNA Polymerase (Finnzymes; Catalog No. F-530L). All PCRs were performed according to the Finnzymes protocol supplied with the polymerase. The PCR conditions for the primary PCRs were:
For primary PCR 1:

pUB-BglII-FW primer and a specific NPRE reverse mutagenesis primer- both 1 μL (10 μM) ; For primary PCR 2:
pUB-BglII -RV primer and a specific NPRE forward mutagenesis primer - both 1 μL (10 μM) ; together with

| | |
|---|---|
| 5 x Phusion HF buffer | 10 μL |
| 10 mM dNTP mixture | 1 μL |
| Phusion DNA polymerase | 0.75 μL (2 units/ μL) |
| DMSO, 100% | 1 μL |
| pUBnprE template DNA | 1 μL (0.1 - 1 ng/ μL) |
| Distilled, autoclaved water | up to 50 μL |

The PCR program was: 30 seconds at 98°C, 30x (10 seconds at 98°C, 20 seconds at 55°C, 1.5 minute at 72°C) and 5 min at 72°C, performed in a PTC-200 Peltier thermal cycle (MJ Research). The PCR experiments resulted in two fragments of approximately 2 to 3 kB, which had about 30 nucleotide base overlap around the NprE mature codon of interest. Fragments were fused in a third PCR reaction using these two aforementioned fragments and the forward and reverse *Bgl*I primers. The fusion PCR reaction was carried out in the following solution: pUB-BglII-FW primer and pUB-BglII-RV primer - both 1 μL (10 μM)
together with

| | |
|---|---|
| 5 x Phusion HF buffer | 10 µL |
| 10 mM dNTP mixture | 1 µL |
| Phusion DNA polymerase | 0.75 µL (2 units/ µL) |
| DMSO, 100% | 1 µL |
| primary PCR 1 reaction mix | 1 µL |
| primary PCR 2 reaction mix | 1 µL |
| Distilled, autoclaved water | up to 50 µL |

The PCR fusion program was as follows: 30 seconds at 98°C, 30x (10 seconds at 98°C, 20 seconds at 55°C, 2:40 minute at 72°C) and 5 min at 72°C, in a PTC-200 Peltier thermal cycler (MJ Research). The amplified linear 6.5 Kb fragment was purified using the QIAQUICK® PCR purification kit (Qiagen, Catalog No. 28106) and digested with *Bgl*II restriction enzyme to create cohesive ends on both sides of the fusion fragment:

- 35 µL purified linear DNA fragment
- 4 µL REACT® 3 buffer (Invitrogen)
- 1 µL *Bgl*II, 10 units/ml (Invitrogen)

Reaction conditions: 1 hour, 30°C.

Ligation of the *Bgl*II digested and purified using QIAQUICK® PCR purification kit (Qiagen, Catalog No. 28106) fragment resulted in circular and multimeric DNA containing the desired mutation:

- 30 µL of purified *Bgl*II digested DNA fragment
- 8 µL T4 DNA Ligase buffer (Invitrogen Catalog No. 46300-018)
- 1 µL T4 DNA Ligase, 1 unit/µL (Invitrogen Catalog No. 15224-017)

Reaction conditions: 16-20 hours, at 16°C.

Subsequently, the ligation mixture was transformed into a *B. subtilis* (Δ*aprE*, Δ*nprE*, *oppA*, Δ*spoIIE*, *degUHy32*, Δ*amyE::(xylR,pxylA-comK)* strain. Transformation to *B. subtilis* was performed as described in WO 02/14490. For each library, 96 single colonies were picked and grown in MOPS media with neomycin and 1.25 g/L yeast extract for sequence analysis (BaseClear) and screening purposes. Each library included a maximum of 19 nprE site-specific variants.

The variants were produced by growing the *B. subtilis* SEL transformants in 96 well MTP at 37°C for 68 hours in MBD medium with 20 mg/L neomycin and 1.25 g/L yeast extract.

## EXAMPLE 4

### Generation of Variant Proteases via QUIKCHANGE® Mutagenesis

In this Example, alternative methods to generate *nprE* SELs are described, although the methods provided herein are suitable for production of SELs of other enzymes of interest (e.g., *Asp)*. As in Example 3, above, the pUBnprE vector containing the nprE expression cassette, served as the template DNA source for the generation of *nprE* SELs and NprE variants. The major difference between the two methods is that this method requires amplification of the entire vector using complementary site-directed mutagenic primers.

Materials:

*Bacillus* strain containing the pUBnprE vector
Qiagen Plasmid Midi Kit (Qiagen Catalog No. 12143)
Ready-Lyse Lysozyme (Epicentre Catalog No. R1802M)
dam Methylase Kit (New England Biolabs Catalog No. M0222L)
Zymoclean Gel DNA Recovery Kit (Zymo Research Catalog No. D4001)
nprE site-directed mutagenic primers, 100nmole scale, 5' phosphorylated, PAGE purified (Integrated DNA Technologies)
QUIKCHANGE® Multi Site-Directed Mutagenesis Kit (Stratagene Catalog No. 200514) MJ Research PTC-200 Peltier Thermal Cycler (Bio-Rad Laboratories)
1.2% agarose E-gels (Invitrogen Catalog No. G5018-01)
TempliPhi Amplification Kit (GE Healthcare Catalog No. 25-6400-10)
Competent *B. subtilis* cells (Δ*aprE*, Δ*nprE*, *oppA*, Δ*spoIIE*, *degUHy32*, Δ*amyE::(xylR,pxylA-comK)*)

Methods:

To obtain the pUBnprE plasmids containing one mutation (identified through *nprE* SEL screening as described above in Example 4 and in US Pat. Appln Ser. No. 11/581,102), a single colony of each *Bacillus* strain of interest was used to inoculate a 5ml LB + 10 ppm neomycin tube (e.g., starter culture). The culture was grown at 37°C, with shaking at 225 rpm for 6 hours. Then, 100 ml of fresh LB + 10ppm neomycin were inoculated with 1ml of the starter culture. This culture was grown overnight at 37°C, with shaking at 225 rpm. Following this incubation, the cell pellet was harvested by sufficient centrifugation to provide a cell pellet. The cell pellet was resuspended in 10 ml Buffer P1 (Qiagen Plasmid Midi Kit). Then, 10μl of Ready-Lyse Lysozyme was added to the resuspended cell pellet and incubated at 37°C for 30 min. The Qiagen Plasmid Midi Kit protocol was continued using 10 ml of Buffer P2 and P3 to account for the increased volume of cell culture. After isolation from *Bacillus* of each pUBnprE plasmid containing a single nprE mutation, the concentration of each plasmid was determined. The plasmids were then *dam* methylated using the *dam* Methylase Kit (New England Biolabs) per the manufacturer's instructions, to methylate approximately 2 μg of each pUBnprE plasmid per tube. The Zymoclean Gel DNA recovery kit was used to purify and concentrate the *dam*-methylated pUBnprE plasmids. The dam-methylated pUBnprE plasmids were then quantitated and diluted to a working concentration of 50 ng/μl for each. Mixed site-directed mutagenic primers were prepared separately for each reaction. For example, using pUBnprE T14R plasmid as the template source, the mixed site-directed mutagenic primer tube would contain 10 μl of nprE-S23R, 10 μl nprE-G24R, 10 μl nprE-N46K, and 10 μl nprE-T54R (all primers at 10 μM each). A PCR reaction using the QuikChange Multi Site-Directed Mutagenesis Kit (Stratagene) was performed following the manufacturer's instructions (*e.g.,* 1 μl dam methylated pUBnprE plasmid containing one mutation (50 ng/μl), 2 μl nprE site-directed mutagenic primers (10 μM), 2.5 μl 10x QuikChange Multi Reaction buffer, 1 μl dNTP Mix, 1 μl QuikChange Multi enzyme blend (2.5U/μl), and 17.5 μl distilled, autoclaved water, to provide a 25 μl total reaction mix. The nprE variant libraries were amplified using the following conditions: 95°C, for 1 min. (1st cycle only), followed by 95°C for 1 min, 55°C for 1 min, 65°C for 13.5 min, and repeat cycling 29 times. The reaction product was stored at 4°C overnight. Then, the reaction mixture underwent *Dpn*I digestion treatment (supplied with QUIKCHANGE® Multi Site-Directed Mutagenesis Kit) to digest parental pUB-nprE plasmid, using the manufacturer's protocol (*i.e.,* 1.5 μl *Dpn*I restriction enzyme was added to each tube and incubated at 37°C for 3 hours; 2 μl of *Dpn*I-digested PCR reaction was then analyzed on a 1.2% E-gel to ensure PCR reaction worked and that parental template was degraded. TempliPhi rolling circle amplification was then used to generate large amounts of DNA for increasing library size of the nprE multi variants, using the manufacturer's protocol (*i.e.,* 1 μl DpnI treated QuikChange Multi Site-Directed Mutagenesis PCR, 5 μl TempliPhi Sample Buffer, 5 μl TempliPhi Reaction Buffer, and 0.2 μl TempliPhi Enzyme Mix, for an ~11 μl total reaction; incubated at 30°C for 3 hours; the TempliPhi reaction was diluted by adding 200 μl distilled, autoclaved water and briefly vortexed. Then, 1.5 μl of diluted TempliPhi material was transformed into competent *B. subtilis* cells, and nprE multi variants were selected for using LA + 10 ppm Neomycin + 1.6 % skim milk plates. Colonies were picked and then sequenced to identify the different nprE variant library combinations.

Table 4-1 provides the primer name, and sequence used in these experiments. Integrated DNA Technologies synthesized all of the primers (100 nmole scale, 5'-phosphorylated, and PAGE purified). Additional mutagenesis primers are described in US Pat. Appln. Ser. No. 11/581,102). Sites evaluated included: 4, 12, 13, 23, 45, 49, 50, 54, 59, 60, 65, 82, 90, 110, 119, 128, 129, 130, 135, 136, 137, 138, 139, 140, 151, 152, 155, 179, 190, 197, 198, 199, 204, 205, 214, 216, 217, 218, 219, 220, 221, 222, 224, 243, 244, 260, 261, 263, 265, 269, 273, 282, 285, 286, 289, 293, 296, 297 and 299.

| Table 4-1. nprE Primers | |
|---|---|
| **PRIMER** | **SEQUENCE** |
| nprE-T14R | GGT ACG ACT CTT AAA GGA AAA AGA GTC TCA TTA AAT ATT TCT TCT GAA AG (SEQ ID NO:8) |
| nprE-S23R | GTC TCA TTA AAT ATT TCT TCT GAA AGA GGC AAA TAT GTG CTG CGC GAT C (SEQ ID NO:9) |

(continued)

| Table 4-1. nprE Primers | |
|---|---|
| **PRIMER** | **SEQUENCE** |
| nprE-G24R | CTC ATT AAA TAT TTC TTC TGA AAG CAG AGG CAA ATA TGT GCT GCG CGA TC (SEQ ID NO:10) |
| nprE-N46K | CAC AAA TTA TTA CGT ACG ATC TGC AAA AAC GCG AGT ATA ACC TGC (SEQ ID NO:11) |
| nprE-T54R | GTA TAA CCT GCC GGG CAG ACT CGT ATC CAG CAC CAC AAA CCA G (SEQ ID NO:12) |

**EXAMPLE 5**

**Expression, Fermentation, Purification and Characterization of Variant Proteases**

This Example describes the methods used to express, ferment and purify the proteases of the transformed *B. subtilis* of the preceding Examples.

Recombinant *Bacillus subtilis* was cultivated by conventional batch fermentation in a nutrient medium. One glycerol vial of *B. subtilis* culture containing the *B. amyloliquefaciens* neutral metalloprotease was used to inoculate 600 ml of SBG 1% medium containing 200 mg/L chloramphenicol. The cultures were grown for 36-48 hours at 37°C, after which time, the culture fluid was recovered by centrifugation at 12,000 rpm, as known in the art. This procedure was done in duplicate. The final enzyme concentrations obtained for 48 hour cultures were in the range of about 1.4 and 2 g/L. After 36 hours of incubation at 37°C, the fermentation broth was recovered and centrifuged at 12,000 rpm (SORVALL® centrifuge model RC5B). The secreted neutral metalloproteases were isolated from the culture fluid and concentrated approximately 10-fold using an Amicon filter system 8400 with a BES (polyethersulfone) 10 kDa cutoff.

The concentrated supernatant was dialyzed overnight at 4°C against 25 mM MES buffer, pH 5.4, containing 10 mM NaCl. The dialysate was then loaded onto a cation-exchange column Poros HS20 (total volume ~ 83 mL; binding capacity ~ 4.5 g protein/mL column; waters) as described below. The column was pre-equilibrated with 25 mM MES buffer, pH 5.4, containing 10 mM NaCl. Then, approximately 200-300 mL of sample was loaded onto the column. The bound protein was eluted using a pH gradient from 5.4 to 6.2 over 10-column volumes of MES buffer. Elution of the protein was between pH 5.8 and 6.0, and was assessed using proteolytic activity as described herein and 10 % (w/v) NUPAGE® SDS-PAGE (Novex). The neutral protease containing fractions were then pooled. Calcium and zinc chloride salts in the ratio of 3:1 were added prior to the adjustment of the pH to 5.8. The Perceptive Biosystems BIOCAD® Vision (GMI) was used for protein purification.

The purified protein, assessed using a 10% (w/v) NUPAGE® SDS-PAGE, was determined to homogenous, with greater than 95% purity. Typically, the purified preparations showed negligible serine protease activity when assessed using the standard protease assay with the substrate, N-succinyl-L-Ala-L-Ala-L-Pro-L-Phe-*p*-nitroanilide (Bachem). This assay was performed in microtiter plate (MTP) format (96 well) using a 100 mM Tris-HCl buffer, pH 8.5, containing 10 mM $CaCl_2$ and 0.005 % TWEEN®-80. The substrate (p-AAPF NA) was prepared by making a 160 mM stock in DMSO (dimethylsulfoxide) (100 mg/ml) and diluting this stock 100-fold with the Tris-HCl buffer containing $CaCl_2$ and 0.005 % TWEEN®-80. Then 10 $\mu$L of diluted protease solution (dilutions were prepared using 100 mM Tris-HCl buffer, pH 8.5, containing 10 mM $CaCl_2$ and 0.005 % TWEEN®-80) was added to 190 $\mu$L 1mg/ml p-AAPF NA solution. The assay was mixed for 5 minutes and the kinetic change at 410 nm was read over 2 to 5 minutes. The slope of the response was measured and used as an indication of the amount of serine protease activity. The protein was formulated for storage using 25 mM MES buffer, pH 5.8, containing 1 mM zinc chloride, 4 mM calcium chloride, and 40 % propylene glycol.

**EXAMPLE 6**

**Balancing Mutational Effects On Protease Activity and Stability**

This Example describes multiply-substituted protease variants engineered to optimize two conflicting enzyme properties. Table 6-1 shows how the charge change was calculated:

| Table 6-1. Calculation of Charge Change | | |
|---|---|---|
| Wild-type Amino Acid | Mutant Amino Acid | Charge Change |
| Neutral | Neutral | 0 |
| Positive | Neutral | -1 |
| Negative | Neutral | +1 |
| Neutral | Positive | +1 |
| Positive | Positive | 0 |
| Negative | Positive | +2 |
| Neutral | Negative | -1 |
| Positive | Negative | -2 |
| Negative | Negative | 0 |

As determined during development of the present invention, the median stability decreased with increasing positive charge. However, BMI cleaning performance increased with increasing positive charge. An optimum BMI cleaning performance under the tested conditions was achieved with a charge change of about +1.

Enhanced stability and BMI cleaning performance are desirable in an engineered variant of NprE. These properties, however, are apparently conflicting. As determined during development of the present invention, using the methods of the present invention, it is possible to produce a more stable variant without severely compromising BMI cleaning performance by selectively combining single mutations. The strategy described herein was successfully used to produce multiply-substituted NprE variants having improvements in a first property (*e.g.*, stability as the primary property), while improving or not sacrificing a second property (e.g., BMI cleaning performance as the secondary property). In particular, the following criteria were employed to select substitutions of interest. The mutations that provide elevated detergent stability or BMI cleaning performance without unduly sacrificing the other parameter were selected. In addition, the charge mutations were balanced, so the final variant is +1 to +3 relative to the wild-type enzyme. In addition, amino acid residues that appeared to be non-interacting in the 3-D structure, to minimize non-additivity between multiple mutations.

During the development of the present invention, four variants were constructed, each containing from ten to eighteen substitutions. These variants are shown in Table 6-3. Importantly, these multiply-substituted variants have increased detergent stability and similar cleaning performance as compared to the wild-type enzyme. This was accomplished by introducing negative and neutral charge stability mutations that were not highly detrimental to BMI cleaning performance, with balancing positive charge performance mutations that did not unduly affect stability. An additional set of pairs of variants were constructed. The first of each pair has a stabilizing negative charge mutation which decreased BMI cleaning performance, and the second of each pair has a compensating positive charge mutation which restored BMI cleaning performance while maintaining stability above the wild-type level. The cleaning performance and stability values for these variants are also in Table 6-4.

| Table 6-3. Multi-Site NprE Variants | | |
|---|---|---|
| Variant | Charge Change | Multiple Substitutions |
| | | |
| 18AA | +3 | 4K-45K-50R-54K-59K-90K-129I-138L-179P-190L-199E-214Q-220E-244S-265P-269H-285R-296E |
| 14AA | +2 | 45K-50R-59K-90K-129I-138L-179P-190L-199E-214Q-220E-244S-265P-285R |
| 12AA | +2 | 45K-59K-90K-1291-138L-179P-190L-199E-214Q-220E-265P-285R |
| 10AA | +1 | 59K-90K-1291-179P-190L-199E-214Q-220E-265P-285R |

| Table 6-4 Stability and Cleaning Performance of NprE Variants | | | |
|---|---|---|---|
| NprE variant | Charge Change | Relative Microswatch Performance | Residual Activity in TIDE® |
| Wild type | 0 | 1.00 | 22.8 |
| S199E | -1 | 0.82 | 54.4 |
| Q45K S199E | 0 | 1.14 | 57.0 |
| K269T | -1 | 1.01 | 44.9 |
| G24K K269T D220E | 0 | 1.11 | 63.0 |
| R280L | -1 | 0.96 | 39.2 |
| T4K R280L | 0 | 1.14 | 41.0 |
| K244S | -1 | 0.89 | 47.2 |
| S23K K244S | 0 | 1.07 | 53.6 |
| K214Q | -1 | 0.81 | 62.1 |
| N90K K214Q | 0 | 0.99 | 56.3 |
| 10AA | +1 | 0.91 | 102.0 |
| 12AA | +2 | 0.55 | 104.0 |
| 14AA | +2 | 0.71 | 101.3 |
| 18AA | +3 | 0.40 | 94.6 |

## EXAMPLE 7

### Balancing Mutational Effects on Amylase Activity and Expression

This Example illustrates that two conflicting enzyme properties can be simultaneously optimized by the introduction of multiple amino acid substitutions.

In this Example, experiments conducted to produce *Bacillus stearothermophilus* alpha amylase (also referred to herein as AmyS), a mutant truncated form of AmyS (S242Q having a 29 amino acid deletion, also referred to herein as S242Q) variants thereof in *B. subtilis* are described. Transformation was performed as known in the art (*See e.g.,* WO 02/14490). Briefly, the gene encoding the parent amylases was cloned into the pHPLT expression vector, which contains the LAT promoter (PLAT), a sequence encoding the LAT signal peptide (preLAT), followed by PstI and HpaI restriction sites for cloning.

### Generation of *B. stearothermophilus* AmyS-S242Q CCL

AmyS-S242Q plasmid DNA was isolated from a transformed *B. subtilis* strain (gentotype: ΔaprE, ΔnprE, amyE::xylRPxylAcomK-phleo) and sent to DNA2.0 Inc. as the template for CCL construction. A request was made to DNA2.0 Inc. (Mountain View, CA) for the generation of positional libraries at each of the four sites in AmyS-S242Q (S242Q) amylase. Variants were supplied as glycerol stocks in 96-well plates. The AmyS S242Q combinatorial charge library was designed by identifying the following four residues: Gln-97, Gln 319, Gln 358, and Gln 443. A four site, 81-member CCL was created by making all combinations of three possibilities at each site: wild-type, arginine, or aspartic acid.

The amino acid sequence of the mature truncated S242Q amylase with the substituted amino acid shown in italics was used as the basis for making the variant libraries described herein:

AAPFNGTMMQYFEWYLPDDGTLWTKVANEANNLSSLGITALWLPPAYKGTSRSDVGY
GVYDLYDLGEFNQKGTVRTKYGTKAQYLQAIQAAHAAGMQVYADVVFDHKGGADG
TEWVDAVEVNPSDRNQEISGTYQIQAWTKFDFPGRGNTYSSFKWRWYHFDGVDWDES
RKLSRIYKFRGIGKAWDWEVDTENGNYDYLMYADLDMDHPEVVTELKNWGKWYVN
TTNIDGFRLDAVKHIKF*Q*FFPDWLSYVRSQTGKPLFTVGEYWSYDINKLHNYITKTNGT
MSLFDAPLHNKFYTASKSGGAFDMRTLMTNTLMKDQPTLAVTFVDNHDTEPGQALQS
WVDPWFKPLAYAFILTRQEGYPCVFYGDYYGIPQYNIPSLKSKIDPLLIARRDYAYGTQ
HDYLDHSDIIGWTREGVTEKPGSGLAALITDGPGGSKWMYVGKQHAGKVFYDLTGNR
SDTVTINSDGWGEFKVNGGSVSVWVPRKTT  (SEQ ID NO:13).

**Amylase Expression - 2ml scale**

*B. subtilis* clones containing AmyS, S242Q or AmyTS23t expression vectors were replicated with a steel 96-well replicator from glycerol stocks into 96-well culture plates (BD, 353075) containing 150 μl of LB media + 10 μg/ml neomycin, grown overnight at 37°C, 220 rpm in a humidified enclosure. A 100 μl aliquot from the overnight culture was used to inoculate 2000 μl defined media + 10μg/ml neomycin in 5ml plastic culture tubes. The cultivation media was an enriched semi-defined media based on MOPS buffer, with urea as major nitrogen source, glucose as the main carbon source, and supplemented with 1% soytone and 5 mM calcium for robust cell growth. Culture tubes were incubated at 37°C, 250 rpm, for 72 hours. Following this incubation, the culture broths were centrifuged for 10 minutes at 3000 x g. The supernatant solution was decanted into 15 ml polypropylene conical tubes and 80 μL of each sample were aliquoted into 96 well plates for protein quantitation.

**Amylase Concentration Determination by Antibody Titration**

As described herein, alpha-amylase concentration and specific activity was determined by titration with an inhibitory polyclonal antibody. Polyclonal antibodies raised to *Bacillus stearothermophilus* alpha-amylase (AmyS) were found to be strongly inhibitory of AmyS and the alpha-amylase from *Bacillus sp.* TS23 (e.g., the binding is tight enough to produce a linear titration of activity loss). Therefore, this antibody can be used to measure enzyme concentration, which in turn is used to calculate specific activity. Briefly, the amount of enzyme inhibition produced by several known concentrations of antibody is measured. From this information, the concentration of antibody required for complete inhibition is extrapolated, which is equivalent to the enzyme concentration in the sample. Alpha-amylase activity and inhibition was measured using the fluorogenic BODIPY-starch assay. The buffer was 50 mM MOPS, pH 7.0, containing 0.005% Tween-80.

A polyclonal antibody directed against purified AmyS was raised in a rabbit and purified by standard methods. An empirical "apparent concentration" value of an antibody stock solution was determined by measuring the inhibition of a sample of AmyS of known specific activity. Then the antibody sample was used to determine the concentration and specific activity of AmyS and TS23t variants. These values were used to create normalized 96-well enzyme stock plates, where all of the variants were diluted to a common concentration.

**Bodipy-Starch Assays For Determination Of Amylase Activity**

The Bodipy-starch assay was performed using the EnzChek® Ultra Amylase Assay Kit (E33651, Invitrogen). A 1 mg/mL stock solution of the DQ starch substrate was prepared by dissolving the contents of the vial containing the lyophilized substrate in 100 μL of 50mM sodium acetate buffer at pH 4.0. The vial was vortexed for about 20 seconds and left at room temperature, in the dark, with occasional mixing until dissolved. 900 μL of assay buffer (50 mM sodium acetate with 2.6 mM CaCl$_2$ pH 5.8) was added and the vial vortexed for about 20 seconds. The substrate solution was stored at room temperature, in the dark, until ready to use or at 4°C. For the assay, a 100 μg/mL of working solution of the DQ substrate was prepared from the 1 mg/mL substrate solution in the assay buffer. 190 μL of 100 μg/mL substrate solution was added to each well in a 96-well flat-bottom microtiter plate. 10 μL of the enzyme samples were added to the wells, mix for 30 seconds using a thermomixer at 800 rpms. A blank sample that contains buffer and substrate only (no-enzyme blank) was included in the assay. The rate of change of fluorescence intensity was measured (excitation: 485 nm, emission: 520 nm) in a fluorescence microtiter plate reader at 25°C for 5 minutes.

**Alpha-amylase Binding**

Amylase variants were incubated with or without CS-28 rice starch microswatches under standard wash conditions for 30 min. The amount of free enzyme was measured by the BODIPY-starch assay. The fraction of enzyme bound to the microswatches was calculated as follows: Fraction bound = (Activity of enzyme in absence of swatch - Activity of enzyme in presence of swatch)/(Activity of enzyme in absence of swatch)

**Results**

As determined during development of the present invention, the median expression of AmyS-242Q decreased with increasing positive charge. However, specific BODIPY starch hydrolysis increased with increasing positive charge. Enhanced recombinant amylase expression and starch hydrolysis are desirable in an engineered variant of AmyS-242Q suitable for starch liquefaction in the fuel ethanol industry or cleaning in detergent applications for instance. These properties, however, are apparently conflicting properties. As determined during development of the present invention, using the methods of the present invention, it is possible to produce a more highly expressed amylase variant without severely compromising starch hydrolysis by selectively combining single mutations. The strategy described herein was successfully used to produce and select multiply-substituted AmyS-242Q variants having improvements in a first property (*e.g.*, expression as the primary property), while improving or not sacrificing a second property (*e.g.*, starch hydrolysis as the secondary property).

In addition, in converse to median expression of AmyS-242Q variants, rice starch microswatch cleaning increased with increasing positive charge. Enhanced recombinant amylase expression and cleaning performance are desirable in an engineered variant of AmyS-242Q. These properties, however, are also apparently conflicting properties. As determined during development of the present invention, using the methods of the present invention, it is possible to produce a more highly expressed amylase variant without severely compromising cleaning performance by selectively combining single mutations. The strategy described herein was successfully used to produce and select multiply-substituted AmyS-242Q variants having improvements in a first property (*e.g.*, expression as the primary property), while improving or not sacrificing a second property (*e.g.*, rice starch microswatch cleaning as the secondary property).

In particular, an eighty member AmyS-S242Q charge combinatorial library (CCL) comprising variants having combinations of from one to four substitutions of charged residues was tested for shake tube expression, BODIPY-starch hydrolysis, and rice starch cleaning activity. AmyS-S242Q winners are shown in Tables 7-1 and 7-2. Importantly, the multiply-substituted variants of Table 7-1 have equal or improved expression and equal or improved BODIPY-starch hydrolysis as compared to the parent enzyme. Similarly, the multiply-substituted variants of Table 7-2 have equal or improved expression and equal or improved rice starch cleaning activity as compared to the parent enzyme.

| Table 7-1. AmyS-S242Q Expression and BODIPY-Starch Hydrolysis Winners | | | | | | | |
|---|---|---|---|---|---|---|---|
| **Variant** | **97** | **319** | **358** | **443** | **Charge** | **Expression (PI)** | **BODIPY (PI)** |
| 1 | Q97E | Q319E | Q358E | Q443E | -4 | 1.27 | 1.29 |
| 2 | Q97E | Q319E | Q358E | Q443R | -2 | 1.19 | 1.31 |
| 3 | Q97E | Q319E | Q358E | | -3 | 1.00 | 1.43 |
| 4 | Q97E | Q319E | Q358R | Q443E | -2 | 1.23 | 1.43 |
| 7 | Q97E | Q319E | | Q443E | -3 | 1.40 | 1.41 |
| 8 | Q97E | Q319E | | Q443R | -1 | 1.12 | 1.58 |
| 9 | Q97E | Q319E | | | -2 | 1.09 | 1.56 |
| 10 | Q97E | Q319R | Q358E | Q443E | -2 | 1.45 | 1.32 |
| 11 | Q97E | Q319R | Q358E | Q443R | 0 | 1.32 | 1.49 |
| 12 | Q97E | Q319R | Q358E | | -1 | 1.58 | 1.27 |
| 16 | Q97E | Q319R | | Q443E | -1 | 1.09 | 1.51 |
| 17 | Q97E | Q319R | | Q443R | +1 | 1.00 | 1.42 |
| 24 | Q97E | | Q358R | | 0 | 1.08 | 1.14 |
| 25 | Q97E | | | Q443E | -2 | 1.12 | 1.00 |

(continued)

| Table 7-1. AmyS-S242Q Expression and BODIPY-Starch Hydrolysis Winners | | | | | | | |
|---|---|---|---|---|---|---|---|
| Variant | 97 | 319 | 358 | 443 | Charge | Expression (PI) | BODIPY (PI) |
| 64 | | Q319R | Q358E | Q443E | -1 | 1.05 | 1.28 |
| 67 | | Q319R | Q358R | Q443E | +1 | 1.02 | 1.50 |

| Table 7-2. AmyS-S242Q Expression and Rice-Starch Hydrolysis Winners | | | | | | | |
|---|---|---|---|---|---|---|---|
| Variant | 97 | 319 | 358 | 443 | Charge | Expression | CS-28 |
| 1 | Q97E | Q319E | Q358E | Q443E | -4 | 1.27 | 1.01 |
| 11 | Q97E | Q319R | Q358E | Q443R | 0 | 1.32 | 1.18 |
| 12 | Q97E | Q319R | Q358E | | -1 | 1.58 | 1.13 |
| 16 | Q97E | Q319R | | Q443E | -1 | 1.09 | 1.43 |
| 17 | Q97E | Q319R | | Q443R | +1 | 1.00 | 1.55 |
| 24 | Q97E | | Q358R | | 0 | 1.08 | 1.15 |
| 25 | Q97E | | | Q443E | -2 | 1.12 | 1.09 |
| 64 | | Q319R | Q358E | Q443E | -1 | 1.05 | 1.18 |
| 67 | | Q319R | Q358R | Q443E | +1 | 1.02 | 1.15 |

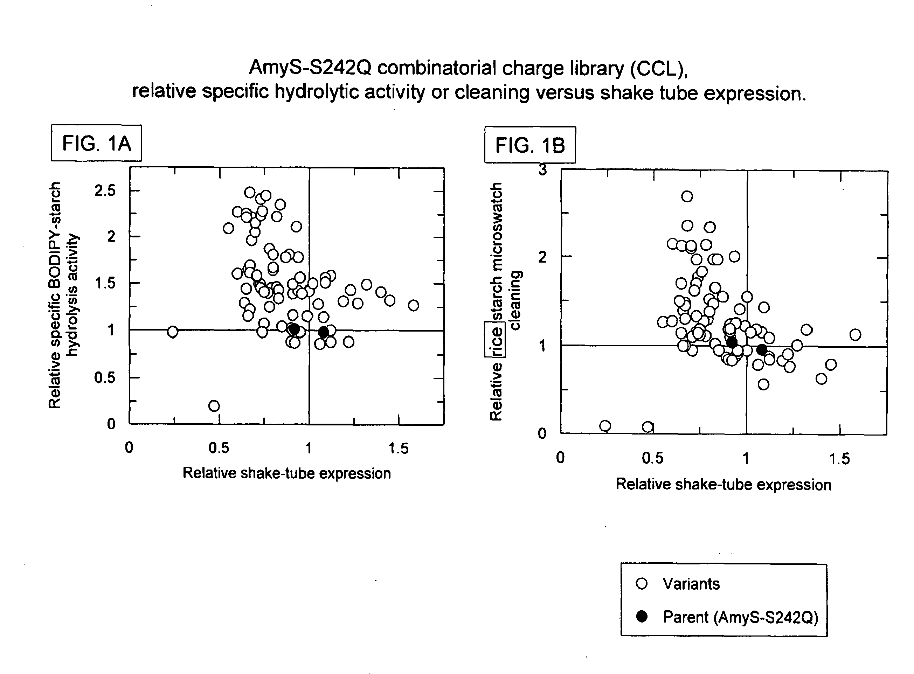

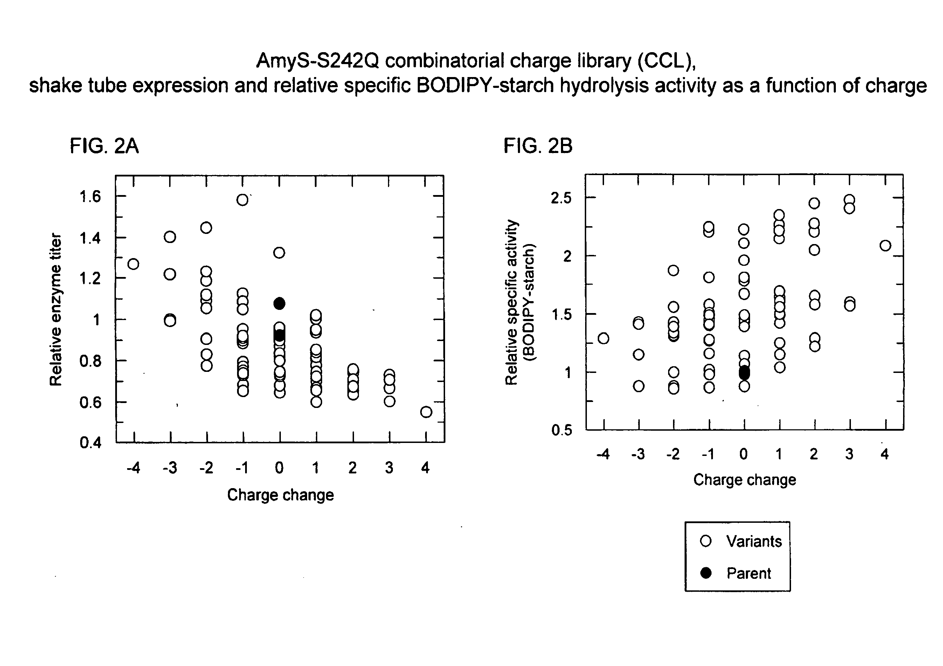

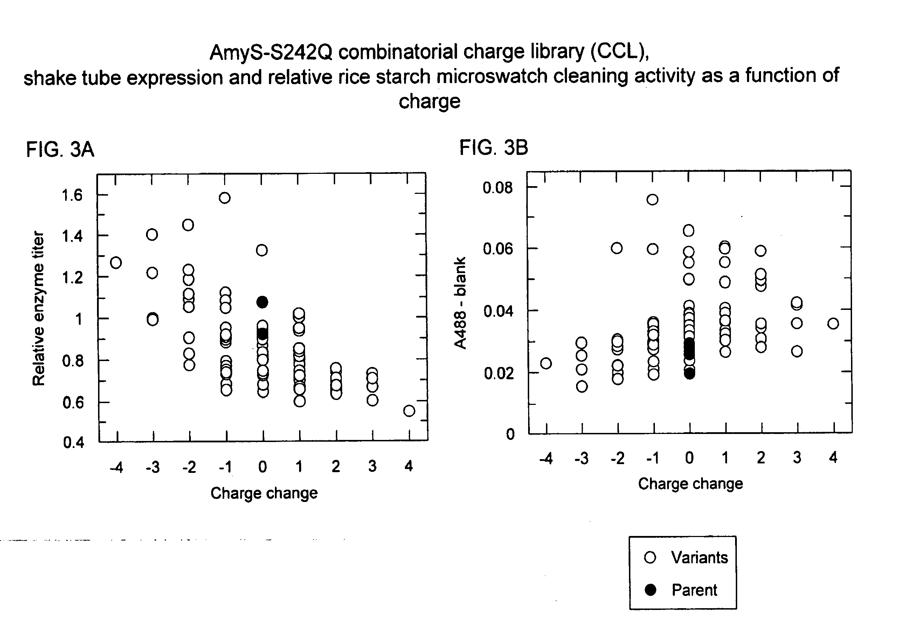

In sum, because enzyme activity and enzyme production have different charge dependences (See FIG. 2A, 2B, 3A and 3B) they are negatively correlated (See FIG. 1A and 1B). However, there are a number of variants that are improved in both expression and activity, and analyzing the library in this manner allows them to be identified.

Although demonstrated with amylases this method is applicable to other enzyme classes such as proteases, lipases, cellulases, transferases, and pectinases. Moreover any combination of two or more properties can be analyzed simultaneously such as expression, activity, binding, thermal stability, detergent and/or chelant stability.

**Claims**

1.   A method for producing improved enzyme variants, comprising in operable order:

   a) testing a plurality of singly-substituted enzyme variants in a first test of a first property and a second test of a second property, wherein the property of a parent enzyme is given a value of 1.0 in each test, a favorable first or second property has a value greater than 1.0, and an unduly unfavorable first or second property has a value less than 0.80;
   b) identifying a substitution in at least one of the singly-substituted enzyme variants that is associated with a favorable first property and which is not associated with an unduly unfavorable second property;
   c) identifying a substitution in at least one of the singly-substituted protein variants that is associated with a favorable second property and which is not associated with an unduly unfavorable first property; and
   d) introducing the substitution from step b) and the substitution from step c) into a protein to yield a multiply-substituted enzyme variant; and
   e) testing the multiply-substituted enzyme variant in the first test and the second test, wherein said multiply-substituted enzyme variant is an improved enzyme variant, and wherein:

      (i) said parent enzyme is a protease, said first property is stability and said second property is wash performance; or
      (ii) said parent enzyme is an amylase, said first property is protein expression and said second property is enzymatic activity,

   and wherein:

at least one of the substitutions comprises a net charge change of 0, -1, or -2 relative to the parent enzyme; and at least one of the substitutions comprises a net charge change of +1 or +2 relative to the parent enzyme thereby defining a charge-balanced multiply-substituted enzyme variant; and

an improved enzyme variant is a variant which achieves a value of greater than 1.0 in both of said first and second tests, or a value of greater than 1.0 in the first test and a value of 0.80 to 1.0 in the second test.

2. The method of claim 1, further comprising producing the improved enzyme variant.

3. The method of claim 1, wherein the first and second properties are negatively correlated.

4. The method of claim 1, wherein said stability is stability in a detergent composition, and wherein said wash performance is blood milk ink (BMI) wash performance.

5. The method of claim 1, wherein said favorable first or second property has a value greater than 1.2.

6. The method of claim 1, wherein said unduly unfavorable first or second property has a value less than 0.60.

7. The method of claim 6, wherein said unduly unfavorable first or second property has a value less than 0.40.

8. The method of claim 1, wherein said wash performance is tested in a powder or liquid detergent composition comprising a pH of between 5 and 12.

9. The method of claim 1, wherein said wash performance is tested in cold water liquid detergent comprising a basic pH.

10. The method of claim 1, wherein said protease is selected from neutral metalloproteases, and serine proteases.

11. The method of claim 10, wherein said serine protease is subtilisin.

12. The method of claim 10, wherein said neutral metalloprotease is a neutral metalloprotease obtained from a member of the family Bacillaceae.

13. The method of claim 12, wherein the neutral metalloprotease is *B. subtilis* NprE.

14. The method of claim 1, wherein said amylase is an alpha amylase obtained from a member of the family Bacillaceae.

15. The method of claim 1, wherein the substitutions are in positions in the parent enzyme having a solvent accessible surface (SAS) of greater than 25%.

16. The method of claim 1, wherein the substitutions are in positions in the parent enzyme having a solvent accessible surface (SAS) of greater than 50% or greater than 65%.

17. The method of claim 1, wherein said parent enzyme is a wild-type enzyme.

18. The method of claim 2, further comprising formulating the improved enzyme variant into a cleaning composition.

**Patentansprüche**

1. Verfahren zur Herstellung verbesserter Enzymvarianten, umfassend in ausführbarer Reihenfolge:

a) das Testen einer Mehrzahl von einfach substituierten Enzymvarianten bei einem ersten Test einer ersten Eigenschaft und einem zweiten Test einer zweiten Eigenschaft, wobei der Eigenschaft eines Elternenzyms bei jedem Test ein Wert von 1,0 zugeteilt wird, eine vorteilhafte erste oder zweite Eigenschaft einen Wert von mehr als 1,0 aufweist und eine unangemessen nachteilige erste oder zweite Eigenschaft einen Wert von wenig als 0,80 aufweist;
b) das Identifizieren einer Substitution in mindestens einer der einfach substituierten Proteinvarianten, die mit einer vorteilhaften ersten Eigenschaft verbunden ist und die nicht mit einer unangemessen nachteiligen zweiten Eigenschaft verbunden ist;

c) das Identifizieren einer Substitution in mindestens einer der einfach substituierten Proteinvarianten, die mit einer vorteilhaften zweiten Eigenschaft verbunden ist und die nicht mit einer unangemessen nachteiligen ersten Eigenschaft verbunden ist; und

d) das Einführen der Substitution aus Schritt b) und der Substitution aus Schritt c) in ein Protein, um eine mehrfach substituierte Enzymvariante zu ergeben; und

e) das Testen der mehrfach substituierten Enzymvariante bei dem ersten Test und dem zweiten Test, wobei die mehrfach substituierte Enzymvariante eine verbesserte Enzymvariante ist und wobei:

(i) das Elternenzym eine Protease ist, die erste Eigenschaft Stabilität ist und die zweite Eigenschaft die Waschleistung ist; oder

(ii) das Elternenzym eine Amylase ist, die erste Eigenschaft die Proteinexpression ist und die zweite Eigenschaft die enzymatische Aktivität ist,

und wobei

mindestens eine der Substitutionen eine Nettoladungsänderung von 0, -1 oder -2 mit Bezug auf das Elternenzym umfasst; und mindestens eine der Substitutionen eine Nettoladungsänderung von +1 oder +2 mit Bezug auf das Elternenzym umfasst, wodurch eine ladungsausgeglichene mehrfach substituierte Enzymvariante definiert wird; und

eine verbesserte Enzymvariante eine Variante ist, die einen Wert von mehr als 1,0 bei beiden des ersten und des zweiten Tests oder einen Wert von mehr als 1,0 beim ersten Test und einen Wert von 0,80 bis 1,0 beim zweiten Test erreicht.

2. Verfahren nach Anspruch 1, ferner das Herstellen der verbesserten Enzymvariante umfassend.

3. Verfahren nach Anspruch 1, wobei die ersten und zweiten Eigenschaften negativ zueinander in Bezug stehen.

4. Verfahren nach Anspruch 1, wobei die Stabilität die Stabilität in einer Detergenszusammensetzung ist und wobei die Waschleistung die Blut-/Milch-/Tinte-(BMT-) Waschleistung ist.

5. Verfahren nach Anspruch 1, wobei die vorteilhafte erste oder zweite Eigenschaft einen Wert von mehr als 1,2 aufweist.

6. Verfahren nach Anspruch 1, wobei die unangemessen nachteilige erste oder zweite Eigenschaft einen Wert von weniger als 0,60 aufweist.

7. Verfahren nach Anspruch 6, wobei die unangemessen nachteilige erste oder zweite Eigenschaft einen Wert von weniger als 0,40 aufweist.

8. Verfahren nach Anspruch 1, wobei die Waschleistung bei einer Pulver- oder Flüssigdetergenszusammensetzung, die einen pH-Wert zwischen 5 und 12 aufweist, getestet wird.

9. Verfahren nach Anspruch 1, wobei die Waschleistung bei einem Kaltwasser-Flüssigdetergens, das einen basischen pH-Wert aufweist, getestet wird.

10. Verfahren nach Anspruch 1, wobei die Protease unter neutralen Metalloproteasen und Serinproteasen ausgewählt wird.

11. Verfahren nach Anspruch 10, wobei die Serinprotease Subtilisin ist.

12. Verfahren nach Anspruch 10, wobei die neutrale Metalloprotease eine neutrale Metalloprotease ist, die von einem Mitglied der Familie Bacillaceae erhalten wird.

13. Verfahren nach Anspruch 12, wobei die neutrale Metalloprotease B. *subtilis* NprE ist.

14. Verfahren nach Anspruch 1, wobei die Amylase eine Alpha-Amylase ist, die von einem Mitglied der Familie Bacillaceae erhalten wird.

15. Verfahren nach Anspruch 1, wobei die Substitutionen sich in Positionen im Elternenzym befinden, die eine für Lösungsmittel zugängliche Oberfläche (LZO) von mehr als 25 % aufweisen.

16. Verfahren nach Anspruch 1, wobei die Substitutionen sich in Positionen im Elternenzym befinden, die eine für Lösungsmittel zugängliche Oberfläche (LZO) von mehr als 50 % oder mehr als 65 % aufweisen.

17. Verfahren nach Anspruch, wobei das Elternenzym ein Wildtyp-Enzym ist.

18. Verfahren nach Anspruch 2, ferner das Formulieren der verbesserten Enzymvariante zu einer Reinigungszusammensetzung umfassend.

**Revendications**

1. Procédé de production de variants améliorés d'enzymes, comprenant dans l'ordre de réalisation:

a) le test d'une pluralité de variants d'enzymes substitués de manière unique dans un premier test d'une première propriété et un second test d'une seconde propriété, la propriété d'une enzyme parente recevant une valeur de 1,0 dans chaque test, une première ou une seconde propriété favorable ayant une valeur supérieure à 1,0, et une première ou une seconde propriété excessivement défavorable ayant une valeur inférieure à 0,80;

b) l'identification d'une substitution au niveau d'au moins l'un des variants d'enzymes substitués de manière unique qui est associée à une première propriété favorable et qui n'est pas associée à une seconde propriété excessivement défavorable;

c) l'identification d'une substitution au niveau d'au moins l'un des variants de protéines substitués de manière unique qui est associée à une seconde propriété favorable et qui n'est pas associée à une première propriété excessivement défavorable; et

d) l'introduction de la substitution de l'étape b) et de la substitution de l'étape c) dans une protéine pour produire un variant d'enzyme substitué de manière multiple; et

e) le test du variant d'enzyme substitué de manière multiple dans le premier test et le second test, où ledit variant d'enzyme substitué de manière multiple est un variant amélioré d'enzyme, et dans lequel:

(i) ladite enzyme parente est une protéase, ladite première propriété est la stabilité et ladite seconde propriété est la performance de lavage; ou

(ii) ladite enzyme parente est une amylase, ladite première propriété est une expression de protéine et ladite seconde propriété est une activité enzymatique,

et où:

au moins l'une des substitutions comprend un changement net de charge de 0, -1, ou -2 par rapport à l'enzyme parente; et au moins l'une des substitutions comprend un changement net de charge de +1 ou +2 par rapport à l'enzyme parente définissant de là un variant d'enzyme substitué de manière multiple à charges équilibrées; et

un variant amélioré d'enzyme est un variant qui atteint une valeur supérieure à 1,0 dans les deux desdits premier et second tests, ou une valeur supérieure à 1,0 dans le premier test et une valeur de 0,80 à 1,0 dans le second test.

2. Procédé selon la revendication 1, comprenant en outre la production du variant amélioré d'enzyme.

3. Procédé selon la revendication 1, dans lequel la première et la seconde propriété sont corrélées de manière négative.

4. Procédé selon la revendication 1, dans lequel ladite stabilité est la stabilité dans une composition détergente, et dans lequel ladite performance de lavage est la performance de lavage sang-lait-encre (BMI).

5. Procédé selon la revendication I, dans lequel ladite première ou seconde propriété favorable a une valeur supérieure à 1,2.

6. Procédé selon la revendication 1, dans lequel ladite première ou seconde propriété excessivement défavorable a une valeur inférieure à 0,60.

**7.** Procédé selon la revendication 6, dans lequel ladite première ou seconde propriété excessivement défavorable a une valeur inférieure à 0,40.

**8.** Procédé selon la revendication 1, dans lequel ladite performance de lavage est testée dans une composition détergente sous la forme d'une poudre ou d'un liquide comprenant un pH compris entre 5 et 12.

**9.** Procédé selon la revendication 1, dans lequel ladite performance de lavage est testée dans un détergent liquide pour eau froide comprenant un pH alcalin.

**10.** Procédé selon la revendication 1, ladite protéase étant sélectionnée parmi les métalloprotéases neutres, et les sérine protéases.

**11.** Procédé selon la revendication 10, dans lequel ladite sérine protéase est la subtilisine.

**12.** Procédé selon la revendication 10, dans lequel ladite métalloprotéase neutre est une métalloprotéase neutre obtenue à partir d'un membre de la famille des Bacillaceae.

**13.** Procédé selon la revendication 12, dans lequel la métalloprotéase neutre est NprE de *B. subtilis.*

**14.** Procédé selon la revendication 1, dans lequel ladite amylase est une alpha amylase obtenue à partir d'un membre de la famille des Bacillaceae.

**15.** Procédé selon la revendication 1, dans lequel les substitutions se trouvent dans les positions dans l'enzyme parente ayant une surface accessible aux solvants (SAS) supérieure à 25 %.

**16.** Procédé selon la revendication 1, dans lequel les substitutions se trouvent dans les positions dans l'enzyme parente ayant une surface accessible au solvant (SAS) supérieure à 50 % ou supérieure à 65 %.

**17.** Procédé selon la revendication 1, dans lequel ladite enzyme parente est une enzyme de type sauvage.

**18.** Procédé selon la revendication 2, comprenant en outre la formulation du variant amélioré d'enzyme en une composition de nettoyage.

AmyS-S242Q combinatorial charge library (CCL),
relative specific hydrolytic activity or cleaning versus shake tube expression.

FIG. 1A

FIG. 1B

EP 2 171 057 B1

○  Variants

●  Parent (AmyS-S242Q)

AmyS-S242Q combinatorial charge library (CCL),
shake tube expression and relative specific BODIPY-starch hydrolysis activity as a function of charge

FIG. 2A

FIG. 2B

O Variants
● Parent

EP 2 171 057 B1

AmyS-S242Q combinatorial charge library (CCL),
shake tube expression and relative rice starch microswatch cleaning activity as a function of
charge

FIG. 3A

FIG. 3B

○ Variants

● Parent

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0479870 B1
- US 6673590 B1
- US 20050221461 A
- WO 9934011 A
- US 6376450 B
- US 8535927 B
- US 4683195 A
- US 4683202 A
- US 4965188 A
- US 5322770 A
- US 6582914 B
- US 6605458 B
- WO 2005052146 A
- WO 0214490 A
- US 581102 A

### Non-patent literature cited in the description

- **RUSSELL ; FERSHT.** *Nature,* 1987, vol. 328, 496-500
- Microbial Proteinases. **KALISZ.** Advances in Biochemical Engineering/Biotechnology. 1988
- **DEL MAR et al.** *Anal Biochem,* 1979, vol. 99, 316-320
- Genetics. **FERRARI et al.** Bacillus. Plenum Publishing Corp, 1989, 57-72
- **GUEROT-FLEURY.** *Gene,* 1995, vol. 167, 335-337
- **PALMEROS et al.** *Gene,* 2000, vol. 247, 255-264
- **TRIEU-CUOT et al.** *Gene,* 1983, vol. 23, 331-341
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad .Sci. USA,* 1988, vol. 85, 2444
- **GAP ; BESTFIT ; FASTA ; TFASTA.** Wisconsin Genetics Software Package. Genetics Computer Group, 1984
- **DEVEREUX et al.** *Nucl. Acid Res.,* 1984, vol. 12, 387-395
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **KARLIN et al.** *Proc. Natl. Acad. Sci., USA,* 1993, vol. 90, 5873-5787
- **ALTSCHUL et al.** *Meth. Enzymol.,* 1996, vol. 266, 460-480
- **KACIAN et al.** *Proc. Natl. Acad. Sci. USA,* 1972, vol. 69, 3038
- **CHAMBERLIN et al.** *Nature,* 1970, vol. 228, 227
- **WU ; WALLACE.** *Genomics,* 1989, vol. 4, 560
- **DYNAN ; TIJAN.** *Nature,* 1985, vol. 316, 774-78
- **GRYCZAN.** *J Bacteriol,* 1978, vol. 134, 318-329
- **NEIDHARDT et al.** *J Bacteriol,* 1974, vol. 119, 736-747